# EUROPEAN PATENT APPLICATION

(11) **EP 2 275 573 A1**
(43) Date of publication of application: **19.01.2011**
(21) Application number: 10150784.6
(22) Date of filing: 30.06.2003
(51) Int. Cl.: C12Q 1/68, A01N 43/04, C07H 21/04, A61K 31/07

(54) **Antibodies for treating and diagnosing gastric adenocarcinoma**

(30) Priority: 28.06.2002 US 392675 P
(62) Divisional of application: 03762257.8
(71) Applicant: Imclone LLC, New York, NY 10014 (US)
(72) Inventor: Pereira, Daniel, Mississauga Ontario L5J 1H4 (CA)
(74) Representative: Ingham, Stephen H.

(57) **Abstract**

The present invention provides antibodies or fragments thereof that bind to S30-21616/DEGA polypeptides for use in the treatment or diagnosis of cancer.

## Description

### FIELD OF THE INVENTION

The present invention relates to polynucleotides and polypeptides that are differentially expressed in a diseased cell and methods of diagnosis, evaluation, treatment, and prevention of diseases relating thereto.

### BACKGROUND OF THE INVENTION

Cancer is the second leading cause of death in the United States, exceeded only by heart disease. In 2002, the American Cancer Society estimated that 1,284,900 new cases will be diagnosed and 550,000 of the 1.6 million living with cancer are expected to die. Cancer is caused by genetic alteration or malfunction of the genes in the cell that results in uncontrolled proliferation of the abnormal cells to form a tumor mass or neoplasm. One of the major factors contributing to uncontrolled proliferation of cells is the over-expression of proto-oncogenes and/or under-expression of tumor suppressor genes. Both these genes play a pivotal role in cancer pathogenesis and the imbalance in polynucleotide expression of these two polynucleotide families perpetuates cancer immortalization. Classes of oncogenes and tumor suppressor genes and polypeptides include growth factors, such as epidermal growth factor (EGF) and transforming growth factor α (TNF-α) and their corresponding receptors, intracellular signaling polypeptides (tyrosine kinases), polynucleotide transcription factors, cell-cycle control polypeptides, and cell-cell or cell-matrix interacting polypeptides.

Uncontrolled cell growth leads to the formation of a localized tumor cell mass, the viability of which is maintained by the formation of neo-vasculature such as blood capillaries that are essential for ferrying nutrients to the cells. Angiogenesis is therefore a key element in the survival, growth, and metastasis of tumors. Polypeptides that are known to play a vital role in angiogenesis are growth factors and their corresponding receptors, including EGF and TGF-α, as well as vascular endothelial growth factor (VEGF).

The localized tumor mass, with its continued rapid cell proliferation, ultimately results in invasion of adjacent tissues as the tumor cells break away from the original tumor mass and migrate to regional lymph nodes and distal vital structures in the body via blood or lymphatic systems. The invasion of tumor cells requires adhesion of cells to the extra-cellular matrix, followed by its breakdown, and finally migration of the tumor cell. The dissemination of certain cancers to preferred sites have been shown to be determined by the presence of certain polypeptides on the cell surface that are capable of homing in on selected cell surface markers on target cells.

Thus, inhibition of uncontrolled cell proliferation, angiogenesis, and cell spread are some of the key targets in the development of therapeutics for managing tumor progression and recurrence. Although much progress has been made in the field of cancer and cancer therapy, many of the therapies in development today has been less than satisfactory, and a need still exists for alternative adjuvant therapy for many of these specific tumor types. The present application involves the discovery of a novel polynucleotide encoding a polypeptide or polypeptide that is differentially expressed in certain tumor cells and can be useful in the diagnosis, evaluation, treatment, and prevention of cancer.

### SUMMARY OF THE INVENTION

The present invention provides isolated and/or purified human and mouse S30-21616/DEGA polynucleotides (*hS30-21616*/*DEGA* or *mS30-21616*/*DEGA* respectively), particularly SEQ ID NO: and SEQ ID NO:3, or a fragment thereof. The present invention also provides isolated and/or purified human and mouse S30-21616/DEGA polypeptides (hS30-21616/DEGA and mS30-21616/DEGA), particularly SEQ ID NO:2 and SEQ ID NO:4, or a fragment thereof. In addition, the present invention provides methods and compositions for diagnosis, evaluation, treatment, and prevention of diseases using such polynucleotides or polypeptides.

### BRIEF DESCRIPTION OF THE DRAWINGS

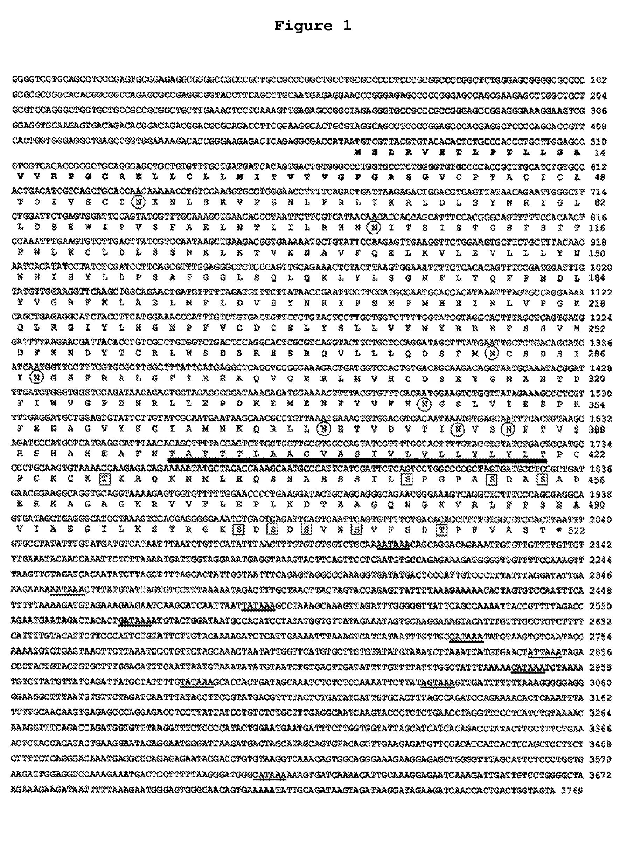

Figure 1 depicts the nucleotide and deduced amino acid sequence of S30-21616/DEGA (cDNA = 3769 bp; ORF = 1569 bp, which encodes a polypeptide of 522 amino acids) with the following features: signal peptide sequence (bold); transmembrane domain (bold underline); putative glycosylation sites (circled amino acids) and putative phosphorylated serines and threonines (boxed amino acids)

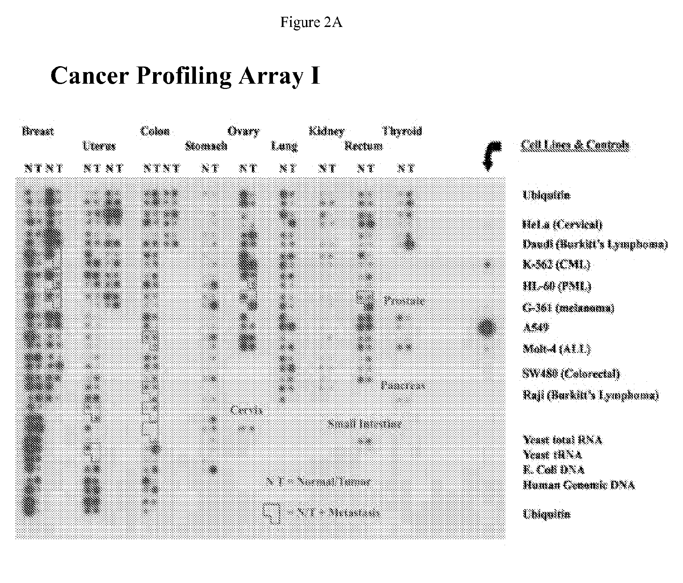

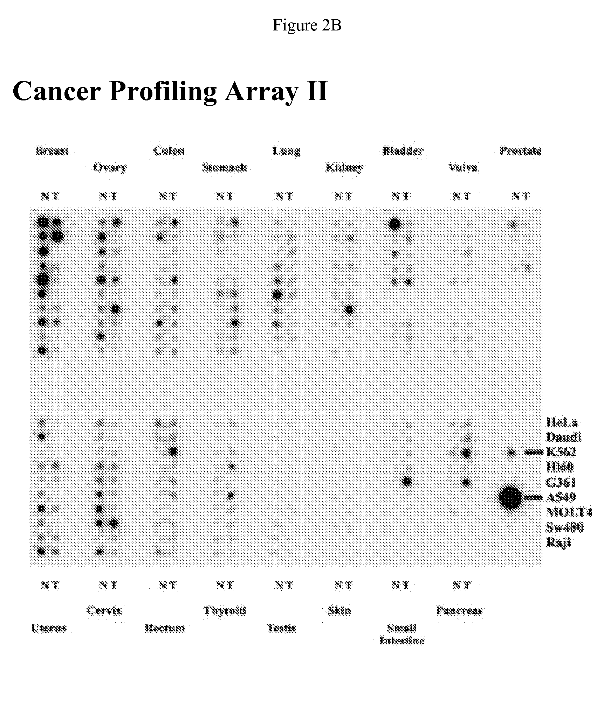

Figure 2 A and B show the dot blot analyses of S30-21616/DEGA cDNA expression levels in tumor and normal tissue samples of individual patients using BD Biosciences Cancer Profiling Arrays I (A) and II (B).

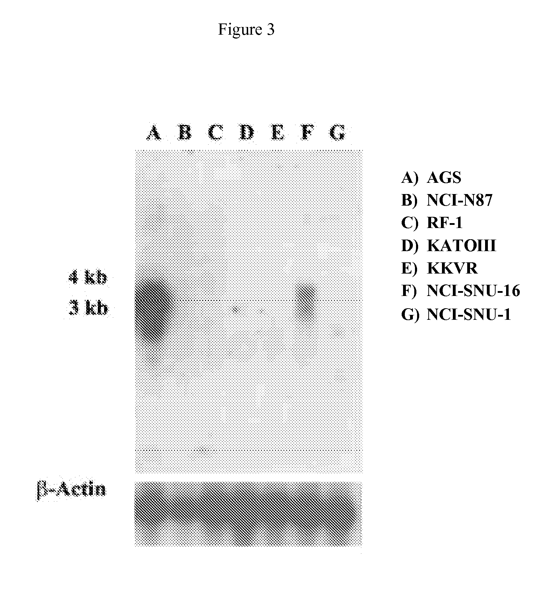

Figure 3 shows the Northern blot analysis of S30-21616/DEGA expression in gastric adenocarcinoma cell lines, AGS (Lane A), NCI-N87 (Lane B), RF-1 (Lane C), KatoIII (Lane D), KKVR (Lane E), NCI-SNU-16 (Lane F), and NCI-SNU-1 (Lane G). The bottom panel shows the mRNA level of β-actin in the respective cell lines.

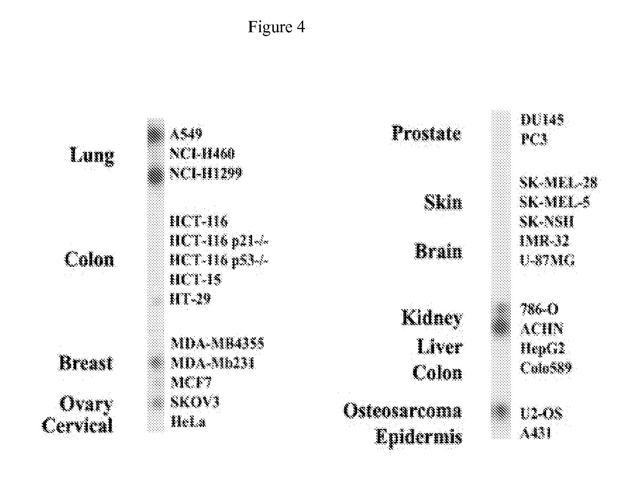

Figure 4 shows the dot blot analysis of S30-21616/DEGA expression in general, non-gastric adenocarcinoma cell lines.

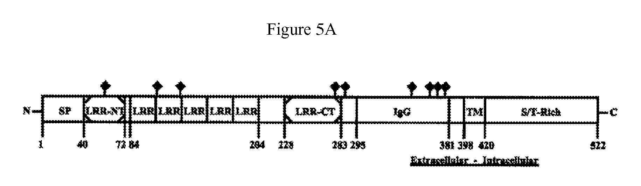

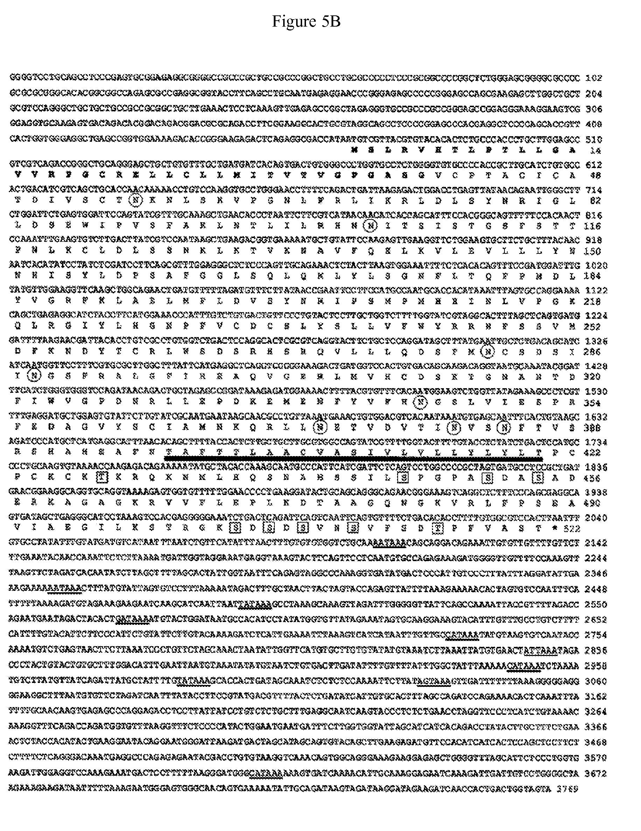

Figure 5A shows the schematic representation the S30-21616/DEGA protein. Domains present in the S30-21616/DEGA protein are shown with their amino acid boundaries as follow: SP=Signal Peptide; LRR=Leucine-Rich Repeat; LRR-NT=Cysteine-rich domain N-terminally flanking LRR; LRR-CT=Cysteine-rich domain C-terminally flanking LRR; IgG=Immunoglobulin domain; TM=transmembrane domain; and S/T-Rich=Serine/Threonine-Rich domain. Black diamonds represent putative N-linked glycosylation sites. Figure 5B shows the amino acid sequence of the S30-21616/DEGA polynucleotide with the LRR bolded, the immunoglobulin-like domain underlined, the transmembrane domain boxed, and the casein kinase phosphorylation and polypeptide kinase C phosphorylation sites indicated by [ ] and ( ), respectively.

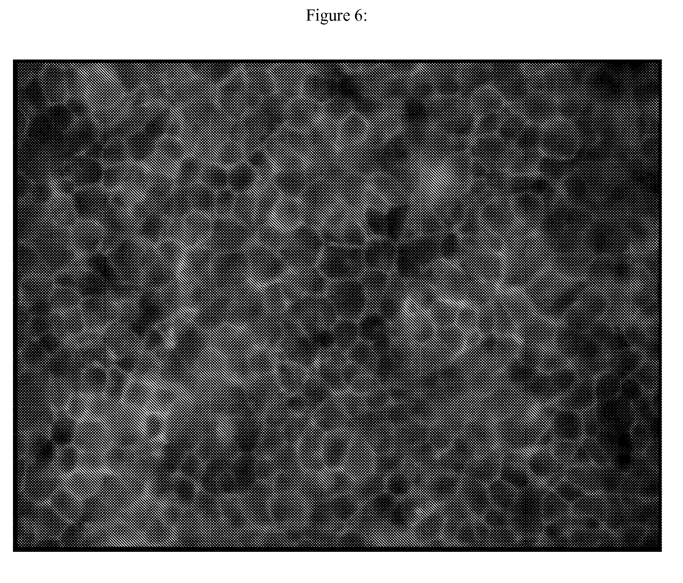

Figure 6 illustrates the sub-cellular localization of the S30-21616/DEGA-EGFP (Enhanced Green Fluorescent Protein) fusion protein that was stably expressed in 293 cells and assessed using fluorescence microscopy (200X magnification).

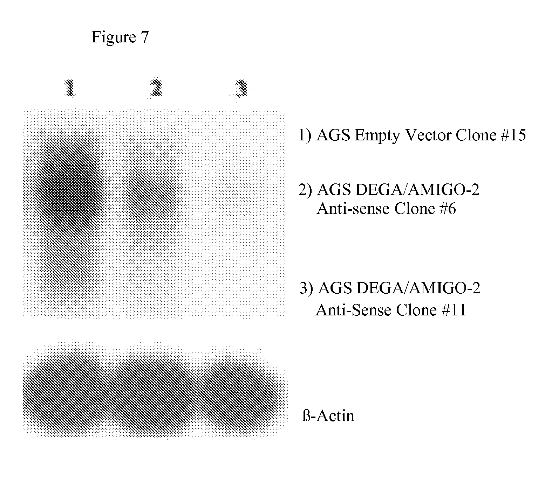

Figure 7 shows the Northern blot analysis S30-21616/DEGA expression in AGS parental or wild type cells (Lane 1), AGS transfected with empty vector, AGS/empty vector clone #15 (Lane 2), AGS transfected with S30-21616/DEGA antisense, AGS/DEGA antisense clone #6 (Lane 3) and AGS/DEGA antisense clone #11 (Lane 4). The bottom panel shows the endogenous expression of β-actin mRNA in the respective cell types (internal control).

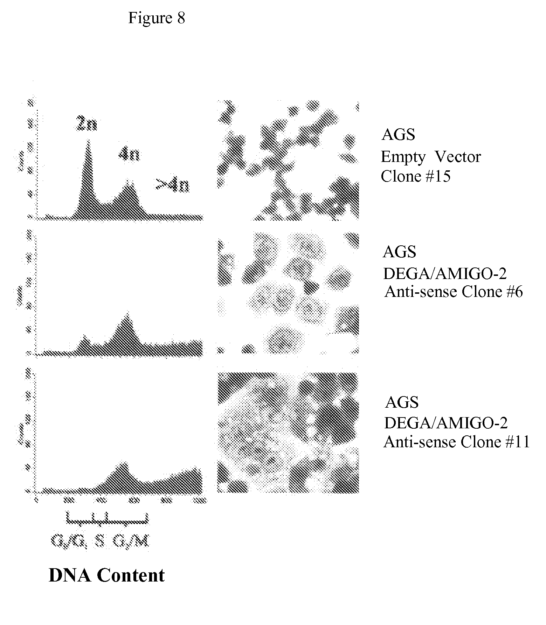

Figure 8 (left panel) shows the flow cytometric histograms of the DNA contents/cell cycle profiles of AGS empty vector clone #15 (top left panel), AGS/DEGA antisense clone #6 (middle left panel) and AGS/DEGA antisense clone #11 (bottom left panel). Figure 8 (right panel) shows the light microscopy comparing the cell size of AGS antisense clone #6 (middle right panel) and clone #11 (bottom right panel) to AGS/empty vector clone #15 (top right panel).

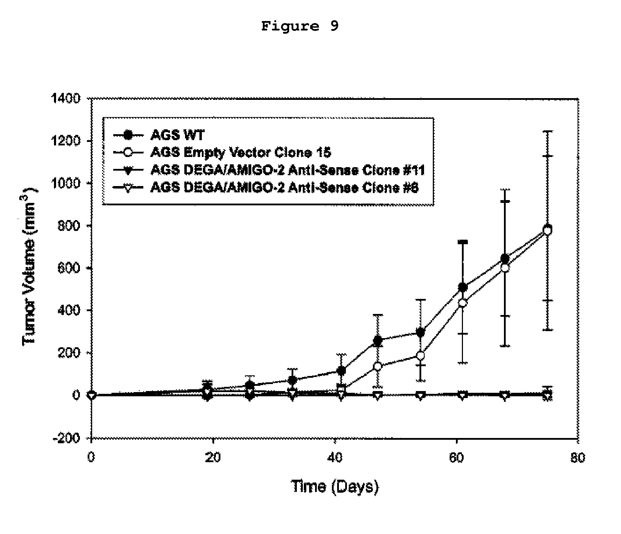

Figure 9 illustrates the tumorigenic growth curves of AGS clones stably transfected with antisense DEGA construct or empty vector determined by the number of tumor-bearing mice versus the total number of mice injected, as follow: AGS WT (12/12); AGS/empty vector clone #15 (19/19); AGS/DEGA antisense clone #6 (6/12); and AGS/DEGA antisense clone #11 (6/19).

### DETAILED DESCRIPTION OF THE INVENTION

The present invention relates to the isolation of novel polynucleotide sequences from a lung carcinoma cell line, A549 cDNA (BD Biosciences/Clonentech, Palo Alto, CA), having a 1569 nucleotide open reading frame. This human nucleotide sequence is referred to as *hS30-21616 or hS30-21616*/*DEGA due to its Differential Expression Profile in Gastric Adenocarcinoma.* The mouse counter-part of this polynucleotide sequence, *mS30-2161*/*DEGA,* which is differentially expressed in a hematopoietic stem cell subtractive cDNA library and in a mouse kidney cancer cell line, has also been isolated. Accordingly, the present invention provides a polynucleotide encoding both a human (hS30-21616/DEGA) and mouse (mS30-21616/DEGA) S30-21616/DEGA polypeptide.

The polynucleotides of the present invention can be DNA, RNA, DNA/RNA duplexes, polypeptide-nucleic acid (PNA), or derivatives thereof. The polynucleotide sequence includes fragments or segments that are long enough to use in polymerase chain reaction (PCR) or various hybridization techniques well known in the art for identification, cloning and amplification of all or part of mRNA or DNA molecules. For example, hybridization under high stringency conditions means the following nucleic acid hybridization and wash conditions: hybridization at 42° C in the presence of 50% formamide; a first wash at 65° C with 2 X SSC containing 1% SDS; followed by a second wash at 65° C with 0.1 X SSC. In addition, the polynucleotides of the present invention include complements of any of the nucleotide or peptides recited above, e.g., cDNA and mRNA.

As used herein, "isolated" or "purified" means that a molecule, e.g., a polynucleotide or polypeptide, is separated from cellular material or other components that naturally accompany it. Typically, the polynucleotide or polypeptide is substantially pure when it is at least 60% (by weight) free from the proteins and other naturally occurring organic molecules with which it is naturally associated. Preferably, the purity of the preparation is at least 75%, more preferably at least 90%, and most preferably at least 99% (by weight) free. A substantially pure polynucleotide or polypeptide can be obtained, e.g., by extraction from a natural source, expression of a recombinant nucleic acid encoding the polypeptide, or chemical synthesis. Purity can be measured by any appropriate method, e.g., column chromatography, polyacrylamide gel electrophoresis, or HPLC analysis. It should be appreciated that the term isolated or purified does not refer to a library-type preparation containing a myriad of other sequence fragments. A chemically synthesized polynucleotide or polypeptide or a recombinant polynucleotide or polypeptide produced in a cell type other than the cell type in which it naturally occurs is, by definition, substantially free from components that naturally accompany it. Accordingly, substantially pure polynucleotides or polypeptides include those having sequences derived from eukaryotic organisms but produced in *E*. *coli* or other prokaryotes.

The isolated nucleic acid or polynucleotides of the present invention preferably have a nucleotide sequence of SEQ ID NO: (*hS30-21616*/*DEGA*) and SEQ ID NO:3 (*mS30-21616*/*DEGA*), or a fragment thereof. Alternatively, and also preferably, the polynucleotides encode a polypeptide with the amino acid sequence of SEQ ID NO:2 (hS30-21616/DEGA) and SEQ ID NO:4 (mS30-21616/DEGA), or a fragment thereof at least eight amino acids in length.

The polynucleotides further include degenerate variants, homologues, or mutant forms of the polynucleotide sequence. By degenerate variant, the term refers to changes in polynucleotide sequences, particularly in the third base of the codon, that do not affect the amino acid sequence encoded by the nucleotide sequences. The term homologue refers to a polynucleotide sequence from a different species having equivalent structure and/or function. Mutant forms refer to alterations of the polynucleotide sequence, such as addition, deletion, or substitution of one or more nucleotides using recombinant DNA techniques well known in the art, or which have been selected naturally. Kunkel et al. (1987) Meth. Enzymol. 154: 367-382-382.

The polynucleotide sequences falling within the scope of this invention include nucleotide sequences that are substantially equivalent to the polynucleotide sequences of SEQ ID NO: or SEQ ID NO: recited above. Polynucleotide sequences of the above invention can have at least about 80%, preferably 90%, and more preferably 95% sequence identity to the polynucleotide recited in SEQ ID NO:1 or SEQ ID NO:3.

The polynucleotides of the present invention encode a polypeptide (S30-21616/DEGA polypeptide). Accordingly, the present invention provides isolated and/or purified S30-21616/DEGA polypeptides. In one embodiment of the present invention, the S30-21616/DEGA polynucleotide encodes an S30-21616/DEGA polypeptide comprising approximately 523 amino acids. In another embodiment, the polypeptide is expressed on the surface of a cell. Preferably, the S30-21616/DEGA polypeptide of the present invention has an amino acid sequence of SEQ ID NO:2 (hS30-21616/DEGA) and SEQ ID NO:4 (mS30-21616/DEGA), or a fragment thereof at least eight amino acids in length.

These polypeptides include functional equivalents, homologues, or mutant forms of the polynucleotide sequence. By functional equivalent, the term refers to alterations in the amino acid sequence, including additions, deletions, and substitutions, that do not substantially alter polypeptide characteristics, e.g., charge, IEF, affinity, avidity, conformation, solubility, and retain the specific function or immunological cross-reactivity of the polypeptide. The term functional equivalents includes conservative amino acid substitutions, which involves a change in the amino acid sequence by way of substituting amino acids of the polypeptide with amino acids having generally similar properties, e.g., acidic, basic, aromatic, size, positively or negatively charged, polarity, non-polarity. The term homologue refers to a polypeptide sequence from a different species having equivalent characteristics and/or function. Mutant forms refer to alterations of the polypeptide sequence, arising due to splicing, polymorphisms, or other events and which may have been selected naturally.

The polypeptide sequences falling within the scope of this invention include amino acid sequences that are substantially equivalent to the polypeptide sequences of SEQ ID NO:2.or SEQ ID NO:4 recited above. Polypeptide sequences of the above invention can have at least about 80%, preferably 90%, and more preferably 95% sequence identity to the polypeptide recited in SEQ ID NO:2 or SEQ ID NO:4.

Preferably, the polypeptides of the present invention have, in a continuous manner, an extracellular N-terminus domain, a transmembrance domain and an intracellular C-terminus domain. *See,* e.g*.,* Figure 5. Such polypeptides include the full length and any extracellular or intracellular variants of the S30-21616/DEGA polypeptide, including, e.g., the soluble variant resulting from deletion of the transmembrane domain (SEQ ID NO:5).

One function of this S30-21616/DEGA polypeptide, although by no means the only function, is a cell surface transmembrane receptor. Transmembrane receptors include, e.g., polypeptide kinases, which are enzymes that catalyze the transfer of a phosphate group from ATP to specific amino acid residues in a target substrate polypeptide. These enzymes are important in a variety of cellular events ranging from signal transduction to membrane transport. Tyrosine polypeptide kinases are involved in mitogenic signaling that initiates rapid signal transduction, while serine/threonine kinases generally integrate and amplify signals.

In addition, S30-21616/DEGA polypeptides may also function as a cell adhesion molecule (CAM). CAMs are a diverse group of glycopolypeptides and carbohydrate molecules that are expressed on the surface of every cell type in unique patterns, depending on the cell type, state of activation of the cell and cell function. These molecules selectively recognize and bind to each other in order to mediate adhesion between cells and also between cells and the extracellular matrix molecules to provide a "VELCRO^{®} effect," allowing cells to explore their environment (see Shimaoka et. al, (2002) Annu. Rev. Biophys. Biomol. Struc. 31:485-516; Wang and Springer (1998) Immunol. Rev. 163:197-215). CAMs are known to play a role in various cellular processes, including, but not limited to, the following processes: (1) organ/tissue development and integrity, (2) initiation and propagation of immune responses, (3) migration or trafficking of immune and inflammatory cells to inflammation sites, (4) wound healing, (5) cancer metastasis, (6) cell signaling, and (7) as selective sites of entry by viral and bacterial pathogen (Cassanova et al. (1998) J. Virol. 72:6244-6246).

Some examples of CAMs include, but are not limited to, selectins, which recognize carbohydrates; integrins, which are primarily expressed on leukocytes and platelets; and members of the immunoglobulin superfamily, which are expressed on both endothelial cells and leukocytes. Members of the immunoglobulin superfamily include intracellular adhesion molecule-1 (ICAM-1), vascular cell adhesion molecule-1 (VCAM-1) and L-1 family of neural adhesion molecules. Binding of CAMs may be calcium dependent or calcium independent. Examples of calcium dependent adhesion molecules include cadhedrins (epithelial E- and P-cadhedrins, desmosomal cadhedrins in epidermis and placenta, and N-cadhedrins found mostly in nerves, muscles and lens cells), selectins, and integrins (also magnesium dependent). Non-calcium dependent CAMs belong to the immunoglobulin (Ig) superfamily. Binding of CAMs to its ligands often trigger the signaling cascade, which involves various kinases such as polypeptide kinase C (PKC), mitogen-activated polypeptide kinase (MAPK), focal adhesion kinase (FAK) and phospholipase C (PLC).

Thus, in one embodiment of the present invention, agents or small molecules capable of disrupting interaction between CAMs and their ligand, or inhibiting the kinases, can be used, for example, to control tumor cell growth. Examples of methods for screening small molecules are described below. In another embodiment of the invention, the nucleotide sequence encoding domain(s) of the polypeptide identified to play a critical role in cell adhesion may be mutated, thereby rendering the molecule incapable of binding to its normal target. Another embodiment of the invention includes engineering such a domain to enhance cell adhesion with various degree of binding to its ligand.

The definition of polypeptide in the present invention also includes within its scope variants or analogs thereof. The term variant is intended to include polypeptides having amino acid additions, including, but not limited to, addition of methionine and/or leader sequences to promote translation and secretion; addition of amino acid sequences or tags to facilitate purification (e.g. polyhistidine sequences); and addition of other polypeptide sequences to produce fusion polypeptides. The term "variant" is also intended to include polypeptides having amino acid deletions of the amino or carboxyl terminus or regions responsible for retention, such as, but not limiting to, the transmembrane region in the polypeptide sequence of S30-21616/DEGA. The present invention includes a polypeptide variant or analog lacking the transmembrane region, resulting in a soluble polypeptide. The term "variant" is also intended to include polypeptides having modifications to one or more amino acid residues that are compatible with the function and structure of the polypeptide. Such modifications include glycosylation, phosphorylation, sulfation and lipidation.

The polypeptide variants or analogs contemplated include polypeptides purified and isolated from a natural source, such as a tumor cell, or recombinantly synthesized; the variant polypeptide having amino acid sequences that differ from the exact amino acid sequence as a result of conservative substitutions, e.g., of the amino acid sequence in SEQ ID NO:2 and SEQ ID NO:4. Conservative amino acid substitutions, as recognized by persons skilled in the art, are changes in amino acid sequence compatible with maintaining the function and structure of the polypeptide.

In a preferred embodiment of the invention, the S30-21616/DEGA polypeptide has a leucine-rich extracellular N-terminus portion, i.e., leucine rich repeats (LRR) of SEQ ID NOS:6, 7, 8, 9, 10, 11 or 12 and an Ig domain of SEQ ID NO:13. *See, e.g.,* Figure 1. Generally, leucine rich polypeptides are known to play a major role in polypeptide-polypeptide interactions in the cell matrix or on the cell surface. The specificity and diversity of the polypeptide-polypeptide interactions may arise from the non-consensus residues flanking the sequence.

Immunoglobulin or Ig-domains, as the term implies, are domains within classes of polypeptide that, similarly to heavy and light chains of immunoglobulins, are well defined regions composed entirely of pairs of β strands or sandwich. These β strands (sheets) or sandwich serve as potential sites for intermolecular recognition. Ig-domains generally function either as structural scaffolds or mediate specific intermolecular interactions with other polypeptides, DNAs, or phospholipids. As structural scaffolds, a series of Ig-domains may serve as building blocks with one domain having intermolecular binding properties and the others interacting with adjacent domains. Examples of polypeptides having Ig-domains as structural scaffolds include the heavy and light chains of immunoglobulins (Amzel & Poljak (1979) Annu. Rev. Biochem. 48:961-997), extracellular domains of T and B cell receptors (Garcia (1999) Annu. Rev. Immunol. 17:369-397), major histocompatibility complexes (Hennecke & Wiley (2001) Cell 104:1-4), growth factors (de Vos et al. (1992) Science 255:306-312), cytokine receptors (Deller & Jones (2000) Curr. Opin. Struc. Biol. 10:213-219) and adhesion molecules (Chothia & Jones (1997) Annu. Rev. Biochem. 66:823-862*).* Preferably, the Ig-domain of the S30-21616/DEGA polypeptide has the amino acid sequence of SEQ ID NO:13. *See, e.g.,* Figure 5B.

In another embodiment of the present invention, the S30-21616/DEGA polypeptide is a soluble polypeptide having at least one LRR and the possibility of an Ig-domain. Preferably, the LRR motifs generally comprise of 20-29 residues that harbor a conserved eleven residue consensus segment (LXXLXLXXLXL), where X can be any amino acid and L can be a leucine, valine, isoleucine or phenylanine residue. Such a polypeptide is at least a monomer; the polypeptide can also be a multimer and preferably a dimer. In another embodiment, the dimer or multimer can be a homodimer or homomultimer. In another preferred embodiment, the dimer or multimer can be a heterodimer or heteromultimer. Alternatively, the S30-21616/DEGA polypeptide is a membrane bound S30-21616/DEGA polypeptide having at least one LRR and an Ig-domain.

The C-terminus domain of the S30-21616/DEGA polypeptide in the present invention has serine and threonine phosphorylation sites that may serve as binding sites for various kinases, e.g., casein kinase II (CKII) and polypeptide kinase C (PKC) phosphorylation sites and may thus function as a signaling receptor. *See, e.g.,* Figure 1. Phosphorylation of either or both the CKII and PKC sites result in intracellular signaling, which often leads to, e.g., uncontrolled cell growth. The present invention includes variants or analogs of the polypeptides having alterations specifically in the C- terminus CKII and PKC sites, such that modulation of phosphorylation and hence signaling can be modulated.

Moreover, the predicted initiating methionine and surrounding sequence (ACCATAATGT) of Figure 1 resembles the Kozak consensus sequence. Potential polyadenylation sequences are underlined. Features of the S30-21616/DEGA protein sequence are as follow: signal peptide sequence (bold); transmembrane domain (bold underline); putative glycosylation sites (circled amino acids) and putative phosphorylated serines and threonines (boxed amino acids).

In one embodiment of the present invention, recombinant constructs are provided. These constructs comprise a S30-21616/DEGA polynucleotide, particularly, SEQ ID NO: and SEQ ID NO:3, or a fragment thereof, and a suitable vector, including, but not limited to, a plasmid or viral vector, phagemid, cosmid, phage vector or derivative useful for expression, replication, probe generation and sequencing purposes. Such constructs can be used to modulate S30-21616/DEGA function.

S30-21616/DEGA function can be up- or down-regulated by modulating the transcriptional or translational control of the nucleic acid in the cell, resulting in inhibition of S30-21616/DEGA polynucleotide expression or altering the amount of S30-21616/DEGA polypeptide. By up-regulation, it is meant that the activity associated with S30-21616/DEGA is enhanced, while down-regulation means diminished or inhibition of the activity. S30-21616/DEGA polynucleotide expression control can be at the level of DNA or RNA. At the DNA level, the functions that can be inhibited includes replication or transcription. The functions of RNA to be interfered with include translocation of RNA to the site of polypeptide translation, translation of polypeptide from the RNA, splicing of RNA to yield one or more mRNA species, and catalytic activity which can be engaged in or facilitated by the RNA.

One agent or construct that can inhibit S30-21616/DEGA polynucleotide expression is an antisense oligonucleotide, which can be in the form of DNA or RNA, or a hybrid thereof. Antisense oligonucleotides can be cDNA, genomic DNA or synthetic RNA or DNA. The DNA can be double-stranded or single stranded, and if single stranded can be the coding or non-coding strand. By antisense, it is referred to specific hybridization of the oligomer with its target nucleic acid, resulting in interference with the normal function of the nucleic acid. All functions of the DNA can be interfered with by antisense, including replication and transcription. Moreover, all functions of the RNA can be interfered with by antisense, including translocation of the RNA to the site of protein translation, translation of protein from mRNA, splicing of the RNA to yield one or more mRNA species, and catalytic activity which can be engaged in or facilitated by RNA, for example. The overall effect of such antisense oligonucleotide is inhibition of expression of S30-21616/DEGA in a cell, which may result in reduced DNA transcription, RNA translation, and polypeptide production.

Preferably, the antisense oligonucleotide has a portion of nucleotide sequence of SEQ ID NO:1 and SEQ ID NO:3, or the complement thereof. Also preferably, the antisense oligonucleotide is complementary to a portion of a nucleotide sequence encoding all or a portion of a S30-21616/DEGA polypeptide of SEQ ID NO:2 and SEQ ID NO:4. Preferably, the antisense oligonuleotide comprises short, gene-specific sequences of S30-21616/DEGA nucleic acid of 20-30 nucleotides in length and more preferably between 12-25 nucleotides in length.

The antisense oligonucleotides of the present invention can be delivered into a cell by any suitable methods, including receptor-mediated endocytosis, microinjection and via DNA-protein complexes or cationic liposomal encapsulation. Another method of improving delivery is by attaching receptor ligands or cell specific antibodies to the antisense oligonucleotide sequences to assist in directing and guiding them to particular target cells and/or tissue regions.

One of the major problems with using antisense oligonucleotide as a therapeutic molecule is the rapid enzymatic degradation of oligonucleotides in blood and in cells. One solution is to terminally inverting the polarity of the oligonucleotide. This concept is based on the assumption that intracellular degradation is mainly due to exonuclease, and consequently, modifications at the ends of the oligonucleotides would stabilize the sequence.

Another proposed solution is via modification of the phosphodiester bonds between nucleotide bases to form, for example, phosphorothioate bonds by substituting one or more non-linking oxygen in the phosphate backbone with sulfur atoms to form, for example, monothiophosphate, and dithiophasphates. These modified oligonucleotides have been shown to have increased half lives. In addition to the sulfur modification, other types of modification in the sugar-phosphate backbone of the anti-sense oligonucleotide include, but not limited to, a 3' amino group substituted for a 3' hydroxyl group in the 2' deoxyribose ring of the DNA (phosphoramidate modification), 2' O-methyl oligo-ribonucleoside phosphodiesters, methylphosphonates (addition of a methyl group to the non-linking oxygen in the phosphodiester backbone), and phopshotriester.

A second generation of anti-sense oligonucleotide include mixed-backbone oligonucleotides which may contain monothiophosphate modification at both the 3' and 5' end of the oligonucleotide. Other examples of modifications include for example, 3'-deoxy-3'-(2-N, N-diphenyliminidazolidino)-thymidine-5'-N,N,-diisopropyl-O-methylphophoramidite, 3'-deoxy-3'-(2-N, N-diphenyliminidazolidino)-thymidine-5'- O-methylphophite. Examples of potential modifiers include for example, but not limited to hydraxine, succinate, ethylenediamine and many others. Alternatively, the 3' position of the unmodified phophosdiester bond can be substituted with a carbon in the P-O linkage to form a 3' methylene or 3'-hydroxymethylene linkage.

One of the side-effects of using antisense oligonuleotides concerns the recognition by the immune system that the antisense oligonucleotide is a foreign molecule, particularly, oligonucleotides that contain the two-base sequence, unmethylated CpG (Cytosine-phosphate-Guanine), which are commonly found in bacteria DNA and not in mammalian DNA. A potential solution is to methylate such an oligonucleotide. Other methods of minimizing the side effects of oligonucleotide treatment involves slow administration of the oligonucleotides in low doses by continuous intravenous injection (instead of administering high doses over a short period of time) and developing topical deliver vehicles that allow for sufficient delivery of the oligonucleotide via the epidermis.

It is also possible to inhibit S30-21616/DEGA expression by inactivating the nucleotide sequence encoding the S30-21616/DEGA polypeptide and hence decreasing or inhibiting polypeptide expression. Such inactivation can occur by deleting the nucleotide sequence in the cell or by introducing a deletion or mutation into the nucleotide sequence, thereby inactivating the nucleotide sequence. The nucleotide sequence can also be inactivated by inserting into the polynucleotide another DNA fragment such that expression of endogenous S30-21616/DEGA does not occur. Methods for introducing mutations, such as deletions and insertions, into genes in eukaryotic cells are well known in the art, e.g., U.S. Patent No. 5,464,764. Oligonucleotides and expression vectors useful for mutation of genes in cells can be made according to methods known in the art and guidance provided herein; for example, methods useful for making and expressing antisense oligonucleotide scan be used to make oligonucleotides and expression vectors useful for mutating genes in cells.

In another embodiment of the present invention, S30-21616/DEGA function or expression can be up-regulated or induced in a cell. Examples of up-regulation include but are not limited to administration to the cell of naked nucleic acids (naked DNA vectors such as oligonucleotides or plasmids), or polypeptides, including recombinantly produced polypeptides. Recombinantly produced polypeptides means that the DNA sequences encoding S30-21616/DEGA are placed into a suitable expression vector, which is described in detail below.

The S30-21616/DEGA polynucleotides of the present invention in a recombinant construct may be operably linked to an expression control sequences or promoters, such control sequences are in known in the art. Kaufman, Meth. Enzymol. (1990) 185:537. The recombinant vector will also include origin of replication to ensure maintenance of vector, one or more selectable markers, leader sequences useful for directing secretion of translated polypeptide into the periplasmic space or extra-cellular medium of host cell.

Targeted mutagenesis and gene replacement of an exogenous human polynucleotide into a non-human mammal (e.g. mouse) provides an *in vivo* research tool for the study and understanding of the biochemical and physiological functions of the polynucleotide. In addition, transgenic non-human mammals also allow for the identification of molecular targets, regulators and therapeutic strategies for the treatment and prevention of diseases or conditions associated with said polynucleotide. Thus, the present invention relates to a transgenic mammals (e.g. mouse) having a non-native nucleotide sequence from another organism inserted into its genome through, e.g., recombinant DNA techniques, which are herein referred to an S30-21616/DEGA transgenics. In addition, the present invention relates to mammals that lack a functional *mS30-21616*/*DEGA* polynucleotide, herein referred to as S30-21616/DEGA knockouts, which can be generated using methods well known in the art. Cappecchi, Science (1989) 244:1288-1292.

In one embodiment of the invention, a conventional knockout non-human mammal is developed, wherein both alleles of the *mS30-21616*/*DEGA* are entirely absent from the cells. In another embodiment of the transgenic non-human mammal, *mS30-21616*/*DEGA* is totally replaced and only the *hS30-21616*/*DEGA* polynucleotide is expressed. In a preferred embodiment of the present invention, a conditional knockout is envisioned, wherein the mS30-21616/DEGA polynucleotide is deleted in a particular organ, cell type, or stage of development. Wagner, Development (2002) 129:1377-1386.

Human S30-21616/DEGA is found to be up-regulated in certain types of tumor cells, such as stomach and thyroid cancers, but generally down-regulated in breast, lung and ovarian cancers. Expression is generally low in normal or non-neoplastic tissue samples and may therefore be useful as a molecular marker for the diagnosis of various cancers. It is therefore an object of this invention that the expression or lack of expression of this polynucleotide can be used to determine the predisposition of an animal, preferably a human subject, of a specific cancer such as, but not limiting to, for example, stomach cancer. Specifically contemplated is a kit, useful in the diagnosis or prognosis of cancer, having a DNA or antisenseDNA or RNA probe derived from the sequences of the S30-21616/DEGA polynucleotide and its variants, in a mixture of reagents well know in the art.

In another embodiment of this invention, the S30-21616/DEGA polynucleotide and/or S30-21616/DEGA polypeptide can be either up-regulated and/or down-regulated. For example, up-regulation or down-regulation of the S30-21616/DEGA on the surface of the cell or secreted into the serum of the patient with cancer can be used diagnostically to confirm or rule out suspected cancer. As a serum or cell surface biomarker, the level of expression of the polypeptide at the time of diagnosis may also provide a more precise prognosis than by staging alone. An immunoassay kit having, e.g., an antibody directed to the polypeptide or a peptide derived from the ligand binding to the polypeptide is envisioned for the present invention. The read-outs for the assays are from standard immunoassays well known in the art. Examples of such assays include, but are not limiting to, enzyme-linked immunosorbent assay (ELISA), radio-immunolabelled assay (RIA), immunoblots and fluorescein-activated cell sorting (FACs).

The invention also discloses a method to purify and produce the full length, extracellular or intracellular variants of the polypeptide. For example, one embodiment of the invention provides recombinant constructs comprising a nucleic acid of SEQ ID NO: and SEQ ID NO:3 having the polynucleotide sequence or fragment thereof, and a suitable expression vector operably linked to a promoter and a leader sequence for expression in a prokaryotic or eukaryotic host cell. Suitable prokaryotic hosts include, for example, *E*. *coli,* such as *E*. *coli HB101, E. coli* W3110, *E. coli* DH1 α; Pseudomonas, Bacillus, such as *Bacillus subtilis* and Streptomyces. Suitable eukaryotic host cells include yeast and other fungi, insect, and animal cells such as CHO cells, COS cells and human cells and plant cells in tissue culture. Accordingly, the present invention provides a cell transfected with an expression construct having an S30-21616/DEGA polynucleotide, e.g., SEQ ID NO:1 and SEQ ID NO:3, operatively linked to expression control sequences. Preferably, such a transfected cell expresses an S30-21616/DEGA polypeptide, e.g., SEQ ID NO:2 and SEQ ID NO:4.

The polypeptides or variants or analogs and fragments thereof of the present invention may be purified by methods well known in the art. For example, the polypeptides or variants and fragments thereof may be expressed as cleavable fusion polypeptide with an appropriate fusion partner that facilitates purification and identification. Useful fusion partner polypeptides include, but are not limited to, histidine tags, glutathione S-transferase or GST, intein [IMPACT™ or Intein Mediated Purification with an Affinity Chitin-binding Tag from New England Biolabs, Beverly, MA; Chong et al. (2001) Gene 275:241:252], and β-galactosidase. The fusion polypeptide may be purified by affinity chromatography using an antibody column, such as an anti-β galactosidase antibody column if β-galactosidase fusion is used.

The polypeptides or variants or analogs thereof in the present invention are useful for the discovery and isolation of one or more putative ligand(s), polypeptide- or DNA-binding partner(s). In another embodiment of the invention, these putative ligand or polypeptide-binding partner, or fragments thereof, may function as inhibitory (antagonist) or stimulatory (agonist) factors as well as in the design of bio-molecules, such as, but not limited to, peptides or antigens and oligonucleotides, or small molecules that are active against S30-21616/DEGA polypeptide of diseased cells, such as, but not limited to, an antagonist. The present invention also provides an S30-21616/DEGA antagonist, which is an agent or compound that is capable of reducing the receptor or receptor-like activity of S30-21616/DEGA. The present invention also provides an S30-21616/DEGA agonist, which is a compound or agent that enhances a desirable pharmacological activity of S30-21616/DEGA.

In a further embodiment of the present invention, these immunopharmaceuticals may be effective in inhibiting the expression, interaction and/or function of this cell surface molecule or its variant forms. These immunopharmaceuticals may be targeted to one or more regions of the polypeptide, such as, but not limited to, the immunoglobulin (Ig)-like domain or LRR of the polypeptide or its signaling pathways represented by, e.g., the polypeptide kinase C, casein kinase II phosphorylation sites, or other phosphorylation sites.

Ligands or small molecules such as an agonist or antagonist that bind to the S30-21616/DEGA polypeptide in the invention can be obtained by any suitable method. As is appreciated by those of skill in the art, there are various such methods to screen for suitable small molecules, examples of, which are described below.

Screening for compounds that bind to, e.g., a cell-line expressing S30-21616/DEGA polypeptide, can be used to identify suitable ligands or small molecules. One such method for screening involves contacting the cells with the compounds to be screened using a microarray type format and determining whether such compounds generate a signal, i.e. activation or inhibition of the polypeptide function, such as but not limited to, a change in signal transduction or pH. These changes are measured to determine if the compound activates or inhibits the polypeptide.

Another method of screening for ligands or small molecules involves the use of the cell line that is loaded with an indicator dye. When bound to the test compound in the presence or absence of an ionic species, such as but not limited to, calcium, a fluorescent signal is produced. The change in fluorescent signal is measured over time using, for example, a fluorescent spectrophotometer or fluorescence imaging plate reader. The change in fluorescence indicates binding of ligand to target polypeptide and that the compound is a potential antagonist or agonist for the polypeptide.

A further method of screening for ligands or small molecules involves the use of a cell line transfected with the polypeptide of the present invention and a reporter polynucleotide construct that is linked to the activation of the polypeptide. Examples of such reporter genes include but are not limited to β-galactosidase, green fluorescent polypeptide (GFP), chloramphenicol acetyltransferase (CAT), luciferase (LUC) and β glucuronidase. After the cells are contacted with the test compound and a known agonist, the signal produced by the reporter polynucleotide over a certain time period can be measured using a fluorimeter, spectrophotometer, luminometer or some other instrument appropriate for the reporter polynucleotide used. A decrease in signal indicates that the compound inhibits the function of the polypeptide and hence is a potential antagonist of the polypeptide.

Another method of screening for compounds that are antagonists, and therefore inhibit activation of the polypeptide, involves binding assays well known in studies on receptorlogy. Cells that express the polypeptide or receptor on the surface of the cell or cell membranes isolated from the cell thereof is contacted with potential antagonist in the presence of a known, labeled ligand. The ligand can be labeled with radionuclides or fluorescent molecules. The amount of labeled ligand bound is measured, i.e. by radioactivity or fluorescent associated with the cell or cell membrane. If the test compound binds to the polypeptide and a reduction of the labeled ligand binding to the receptor is measured, the compound is therefore capable of competing with the natural ligand and hence is a potential antagonist.

Another embodiment of the invention relates to a method for generating or screening of antibodies, especially neutralizing or polypeptide-polypeptide interaction inhibiting antibodies specific for S30-21616/DEGA polypeptides and analogs thereof, antisensemolecules, peptides or small molecules. Antibodies of the present invention include complete anti-S30-21616/DEGA antibodies, as well as antibody fragments and derivatives that comprise an S30-21616/DEGA binding site or the Ig domain and the LRR. Antibody fragments include, but are not limited to, Fab, F(ab')₂, Fv and scFv, dibody or single domain antibody. In another embodiment of the invention, antibodies and fragments thereof to the polypeptide receptor or variants thereof can be selected by phage display library well know in the art (See, e.g., McCafferty et al., (1990) Nature 348:552-554; Aujame et al., (1997) Hum. Antibodies 8:155-168). Combinations of variable domains are typically displayed on filamentous phage in the form of Fab's or scFvs. The library is screened for phage bearing combinations of variable domains having desired antigen-binding characteristics. Preferred variable domain combinations are characterized by high affinity for S30-21616/DEGA. Preferred variable domain combinations can also characterized by high specificity for S30-21616/DEGA and has little cross-reactivity to other related antigens. By screening from very large repertoires of antibody fragments, (2-10x10¹⁰; see e.g., Griffiths et al., (1994) EMBO 13:3245-3260) a good diversity of high affinity monoclonal antibodies can be isolated, with many expected to have sub-nanomolar affinities for S30-21616/DEGA.

The antibody of the present invention may be a monoclonal (MAb) or polyclonal antibody derived from rodents, human or humanized or chimeric antibodies. The major drawback of rodent MAbs is that, although they may be administered to a patient for diagnostic or therapeutic purposes, they are recognized as foreign antigens by the immune system and are unsuitable for continued use. Antibodies that are not recognized as foreign antigens by the human immune system have greater potential for both diagnosis and treatment. Human antibodies are antibodies that consist essentially of human sequences and can be obtained by phage display libraries where combinations of human heavy and light chain variable domains are displayed on the phage surface. Methods for generating human and humanized antibodies are now well known in the art. For a review of antibody humanization and of the nomenclature applied to antibody domains and regions, see, e.g., Vaughan et al. (1998) Nat. Biotechnol. 16:535-539

Chimeric antibodies can be constructed in which regions of a non-human MAb are replaced by their human counterparts. A preferred chimeric antibody is one having the whole variable regions of a non-human antibody such as a mouse or rat variable region expressed along with the human constant region. Methods for producing such antibodies are well known in the art (See, e.g., Jones et al., (1996) Nature 321:522-525; Riechman et al., (1988) Nature 332:323-327, U.S. Patent 5,530,101 and Queen et al (1989) Proc. Natl. Acad. Sci. 86:10029-10033). A more preferred chimeric antibody of the invention is a humanized antibody. A humanized antibody is an antibody in which the complementarity determining region (CDR) of the non-human antibody V-region that binds to, for example, hS30-21616/DEGA, is grafted to the human framework (FW) region (Padlan, (1991) Mol. Immunol. 28:489-498). Amino acid residues corresponding to CDRs and FWs are known to one of skill in the art. Chimeric antibodies can also include antibodies where some or all non-human constant domains have been replaced with human counterparts (see, e.g., LoBuglio et al., (1989) Proc. Natl. Acad. Sci. 86:4220-4224).

In a preferred embodiment of the invention, a therapeutic method for prevention, treatment, or amelioration of a medical condition is disclosed. In this method, the newly isolated antibody, human or humanized antibody and fragments thereof, can be conjugated to a radioisotope such as, but not limited to, Yttrium-90, a cytotoxic or chemotherapeutic agent. The conjugated antibody or fragments thereof can be administered to the patient by injecting in the patient after surgery or chemotherapy an effective amount that is capable of destroying micrometastasis or delaying or preventing recurrence of disease. The antibody and fragments thereof also can be used as a method for diagnosing tumor in the cells of a patient such as a human or an animal.

The antibodies of the present invention are also useful for detecting the present polypeptides and polypeptides in specific tissues or in body fluids. Immunoassays may use a monoclonal or polyclonal antibody reagent that is directed against one epitope of the antigen being assayed. Alternatively, a combination of monoclonal or polyclonal antibodies may be used which are directed against more than one epitope. Protocols may be based, for example, upon competition, direct antigen-antibody reaction or sandwich type assays. Protocols may, for example, use solid supports or immunoprecipitation. The present antibodies can be labeled with a reporter molecule for easy detection. Assays that amplify the signal from a bound reagent are also known. Examples include immunoassays that utilize avidin and biotin or enzyme-labeled antibody or antigen conjugates, such as ELISA assays.

In another preferred embodiment of the invention, a method for eliciting and stimulating an immune response in a human subject in whom prevention, treatment or amelioration of a neoplastic disease is provided. The method includes administering to the subject a composition having an antigenic molecule that is capable of eliciting an immune response, such as an antibody response, and more preferably a cellular immune response. The "antigenic molecule" used herein is a peptide or fragment of a polypeptide that is identified using standard immunoassays known in the art by detecting the ability of said peptide or polypeptide fragment to bind to antibody or MHC molecules (antigenicity) and generate an immune response (immunogenicity). In one aspect, any of the antibodies disclosed herein can be used to elicit and stimulate an immune response, e.g., resulting in death of a cell expressing the S30-21616/DEGA polypeptide. It is further appreciated that the antigenic molecule can be administered as a polynucleotide that is subsequently translated into the antigenic polypeptide *in vivo.* It is also further appreciated that the antigenic molecule is an immunogen having a peptide of at least 8 amino acids.

Any suitable neoplastic disease, i.e., cancer, can be treated using the present inventive methods, including, e.g., stomach, thyroid, breast, ovarian, kidney, or lung cancers.

The antigenic molecule of the therapeutic composition can be administered in a pharmaceutically acceptable carrier or in a non-covalent complex with another polypeptide in the presence or absence of immuno-enhancers or biological response modifiers, including but not limited to, the cytokines such as but not limited to interferons, (IFN-α, IFN γ), interleukins, (IL-2, Il-4, IL-6) or tumor necrosis factors (TNF-α, TNF-β).

Another embodiment of the invention for enhancing an immune response in a human comprises administering to the human subject antigen presenting cells (APCs) sensitized *in vitro* with the antigenic molecule. The APC can be selected from among those antigen presenting cells known in the art, including but not limited to B lymphocytes, dendritic cells, macrophages, T lymphocytes and combination thereof and preferably macrophages and more preferably, dendritic cells.

The polynucleotides of the present invention can be used in the treatment of metastatic cancer. Treatments include polynucleotide ablation, polynucleotide replacement, polynucleotide expression and antisensepolynucleotide suppression. In a preferred embodiment of the invention, the method of treatment of a metastatic cancer, involves administering a pharmaceutical composition containing an effective amount of an S30-21616/DEGA polynucleotide, or fragment thereof, with additional sequences such as promoters for expression of sense and antisensemessage, recombinant sequences for targeting the gene, selectable markers for transfection and selection or replication or origins for passage in a prokaryotic, eukaryotic, bacteria, insect or yeast cells. The nucleic acids used may be single or double stranded DNA, RNA or PNA. The variants of the nucleic acids may include alterations such as deletion, substitution, addition or non-conservative alterations found naturally or engineered.

It is understood that antibodies and antibody conjugates, polynucleotides and fragments, polypeptide and analogs of the invention, small molecules including agonist and antagonist, where used in the human body for the purpose of diagnosis or treatment, will be administered in the form of a composition additionally having a pharmaceutically-acceptable carrier. Such carriers are well known to one of average skill in the art. Modes of administration include, but are not limited to, subcutaneous, intramuscular, intravenous, intraperitoneal, intradermal or mucosal routes.

Human S30-21616/DEGA polynucleotides and polypeptides of this invention are differentially expressed in hematopoietic stem cells (see *Example 2*) and may be useful in the regulation of hematopoiesis. Consequently, the S30-21616/DEGA polynucleotides and polypeptides of this invention, or agents directed against such polynucleotides and polypeptides, may be useful for the treatment of stem cell associated diseases, and in particular disease of hematopoiesis such as myeloid and lymphoid cell deficiencies, for example, leukemia or in supporting the growth and proliferation of erythroid progenitor stem cells. Agonist and antagonist of the polypeptide in this invention are also envisioned in treatment of stem cell associated diseases.

Another embodiment of the invention may be directed to the use of the nucleic acid or polypeptide as marker for stem cell isolation, and in particular in hematopoietic stem cell isolation for use in transplantation. Since hematopoietic stem cells are capable of maturing to various different hematopoietic cells, such as erythroid, myeloid and lymphoid cells. These isolated stems cells may be useful for, but not limited to, repopulation of the stem cells after radiation or chemotherapy, in *in-vivo* or ex-vivo bone marrow transplantation.

The S30-21616/DEGA polynucleotides and polypeptides of the present invention can be administered alone or in combination with other cytokines in a pharmaceutically acceptable composition for treatment of, but not limited to, various anemias or for the use in conjunction with irradiation or chemotherapy to stimulate the production of precursors or erythroid cells; in supporting growth and proliferation of myeloid cells, such as monocytes, macrophages and megakaryocytes; and supporting growth and proliferation of hematopoietic stem cells. In another embodiment of the invention, inhibitory molecules may be used to alter hematopoietic stem cell interacting with supporting stromal cells.

Accordingly, the present invention can be used *in vivo* and *in vitro* for investigative, diagnostic, prophylactic, or treatment methods, which are well known in the art. The examples that follow further illustrate the invention, but should not be construed to limit the scope of the invention in any way. Other embodiments and uses of the invention will be obvious to those skilled in the art from consideration of the specification and practice of the invention disclosed herein. Detailed descriptions of conventional methods, such as those employed in the construction of vectors and plasmids, the insertion of genes encoding polypeptides into such vectors and plasmids, the introduction of plasmids into host cells, and the expression and determination thereof of genes and polynucleotide products, can be obtained from numerous publication, including Sambrook, J. et al., (1989) Molecular Cloning: A Laboratory Manual, 2nd ed., Cold Spring Harbor Laboratory Press. All references mentioned herein are incorporated in their entirety.

### EXAMPLES

### Cell Cultures

Cell lines used were obtained from the American Type Culture Collection (ATCC, Manassas, VA) and media used for their propagation were purchased from Gibco, Grand Island, NY. Cell lines and their respective growth media (shown in parentheses) were as follow: AGS, a human gastric adenocarcinoma cell line₂ (F-12); 293, a human kidney epithelial cell line transformed with adenovirus 5 DNA, (Dulbecco's Modified Eagles Medium (DMEM)); KKVR (Iscove's DMEM); RF-1, a human gastric adenocarcinoma cell line, (Leibovitz's L-15); NCI-SNU-1, NCI-SNU-16, NCI-N87, a human liver gastric carcinoma cell line and KatoIII, a human gastric carcinoma, (RPMI). All media were supplemented with 2 mM L-Glutamine (Gibco, Grand Island, NY) and 10% Fetal Bovine Serum (FBS; Hyclone, Logan, UT). KatoIII and KKVR, however, were grown in 20% FBS and NCI-N87 and KKVR growth media were additionally supplemented with 1.5 g/L sodium bicarbonate.

### Example 1

The present example demonstrates identification of differentially expressed novel cancer genes. Briefly, a murine hematopoietic stem cell subtractive cDNA library was utilized. Phillips et al. (2000) Science 288:1635-1640. Approximately 7,500 ESTs from this library were placed on DNA microarrays and hybridized with cDNA prepared from a variety of murine cancer cell lines and corresponding normal tissue.

One of the murine ESTs, S30-21616/DEGA, was identified as being 7.6 fold differentially expressed in the murine renal cell line, RAG versus normal renal tissue. This EST was sequenced and its 522 bps used to blast the NCBI non-redundant sequence databases. At this time, S30-21616/DEGA showed no homology to any known murine sequences. It did, however, show approximately 85% similarity to human chromosome 12 BAC clones and to a portion of a human endometrial cancer partial cDNA clone (IMAGE: 3625286). The IMAGE: 3625286 DNA sequence was used to generate 5' RACE ORF, and 3' UTR PCR products to give a final cDNA (*hS30-21616*/*DEGA*) of 3769 bp (Figure 1). Transcription of the cDNA resulted in various size transcripts of 2.2 and 3.5 KB, implying alternative splicing.

### Example 2

The present example demonstrates expression of hS30-21616/DEGA in normal and cancer tissues. Briefly, Northern blot analyses using multiple tissue Northern (BD Biosciences/Clonetech, Palo Alto, CA) were performed to assess the expression of *hS30-21616*/*DEGA* in normal and cancer tissues. The normal tissues screened include brain, heart, skeletal muscle, colon, thymus, spleen, kidney, liver, small intestine, placenta, lung, peripheral blood leukocyte, adrenal gland, bladder, bone marrow, lymph node, prostate, spinal cord, stomach, thyroid, trachea, uterus, and tongue. In a majority of these tissues, expression of *hS30-21616*/*DEGA* was very low.

With respect to cancer cell line expression, we assessed *hS30-21616*/*DEGA* expression by Northern blot analysis in HL-60 (Promyelocytic Leukemia), HeLa (Cervical Carcinoma), K562 (Chronic Myeloid Leukemia), Molt-4 (Acute Lymphoblastic Leukemia), Raji and Daudi (Burkitt's Lymphoma), SW480 (Colorectal Adenocarcinoma), G361 (Melanoma), A549 (Lung Carcinoma) cells, AGS (human gastric adenocarcinoma), NCI-SNU-1, NCI-SNU_16, and NCI-N87 (human liver gastric carcinoma).

*Human S30-21616*/*DEGA* was expressed as multiple sized transcripts of 2-3.5 KB in A549 (data not shown) as well as in AGS and NCI-SNU-16 *(see,* Figure 3 PLEASE CHECK, SEE BELOW) cells. These size transcripts are also observed in GenBank^{®} cDNAs expressed in testis, stomach, lung and uterus. Due to its differential expression profile in gastric adenocarcinoma, S30-21616 cDNA is henceforth named *S30-21616*/*DEGA.*

### Example 3

The present example demonstrates Cancer Profiling Array (CPA) analysis of S30-21616/DEGA expression in patient samples using cDNA dot blots. Briefly, in a variety of tumor and normal tissues (CPA) I and II (BD Biosciences Clontech, Palo Alto, CA) were probed with a cDNA fragments encompassing ORF nucleotides 1540-2045 (numbering based on Figure 1) as per manufacturer's recommendations. The probe was labeled using [α-³²P]-dCTP (PerkinElmer Life Sciences, Inc, Boston, MA) and isolated using the Prime-It^{®} II kit (Stratagene Cloning Systems, La Jolla, CA). CPA I and II are nylon arrays containing normalized cDNA from 241 and 160 tumor and corresponding normal tissues obtained from individual cancer patients respectively. Tissues represented by CPA I include: breast, uterus, colon, stomach, ovary, lung, kidney, rectum, thyroid, cervix, small intestine, pancreas and prostate. In addition to these tissues, CPA II carries bladder, testis, and skin tissue. These arrays also contain positive and negative controls as well as cDNA isolated from nine cancer cell lines: HeLa, Daudi, K-562, HL-60, G-361, A549, Molt-4, SW480 and Raji.

The results from the array profile in Figures 2A (CPA 1) and 2B (CPA II) again showed that DEGA was expressed in some normal lung, colon and rectum samples but the expression are generally lower than in the female reproductive tissues. Nearly every patient showed strong expression of DEGA in normal breast tissues. In contrast, DEGA expression in tumor samples was differentially expressed in 56% of thyroid (9/16; 5/6 in CPA I, and 4/10 in CPA II) and 45% of stomach cancers (17/38; 14/28 in CPA I and 3/10 in CPA II). Of the cancer cell lines tested in CPA I (Figure 2A) and CPA II (Figure 2B), A549 expressed high levels of DEGA cDNA and K562 expressing approximately 10 fold less DEGA cDNA than A549 cells. There was minimal expression in Molt-4, G361 and HeLa cells.

Table 1 further provides a summary of the regulated differential expression of DEGA from various tissues using the arrays. The expression levels were normalized against cDNA from 241 tumor and corresponding normal tissues from individual patients.

**TABLE 1.**

| **Type of Cancer** | **Number of Samples** | **Up Regulated** | **Down Regulated** | **No Difference** |
|---|---|---|---|---|
| Stomach | 28 | 45% | 34% | 21% |
| Thyroid | 6 | 83% | 0% | 17% |
| Breast | 50 | 6% | 70% | 24% |
| Ovary | 14 | 0% | 43% | 57% |
| Lung | 21 | 19% | 57% | 24% |
| | | | | |
| Uterus | 42 | 19% | 26% | 55% |
| Colon | 34 | 26% | 26% | 48% |
| Cervix | 1 | 0% | 0% | 100% |
| Kidney | 20 | 10% | 10% | 80% |
| Rectum | 18 | 11% | 11% | 78% |
| Prostate | 4 | 0% | 50% | 50% |
| Pancreas | 1 | 0% | 0% | 100% |
| Small Intestine | 2 | 0% | 0% | 100% |

In twenty-eight of the stomach and six thyroid cancers profiled, *hS30-21616*/*DEGA* was up-regulated in 45% and 83% of tumor respectively versus normal samples. However, in fifty breast, fourteen ovarian, and twenty one lung cancers typed, *hS30-21616*/*DEGA* was down-regulated in 70%, 43% and 57% of tumor respectively versus normal samples. The results showed that *hS30-21616*/*DEGA* is differentially expressed in different tumor types and may play a role in modulating tumor growth.

### Example 4

The present example demonstrates Northern Blot analysis of DEGA expression in gastric adenocarcinoma cells. The observation that 45% of the stomach cancer patient samples showed differential expression of DEGA in their tumors but insignificant expression in adjacent normal stomach tissue suggests that DEGA may play a pivotal role in the development or progression of at least a sub-fraction of gastric adenocarcinoma. Northern Blot analysis was carried out to determine whether DEGA was expressed in the adenocarcinoma cell lines, AGS, NCI-SNU-1, NCI-SNU-16, NCI-N87, KATOIII, KKVR and RF-1.

The autoradiograph in Figure 3 shows that expression in both AGS (Lane A) and NCI-SNU-16 (Lane F) cells, with AGS having eight-fold greater expression over NCI-SNU-16.

### Example 5

The present example demonstrates Cancer Profiling Array (CPA) analysis of S30-21616/DEGA expression in other non-gastric adenocarcinoma cancer cell lines. Using cDNA dot blots, the *S30-21616*/DEGA probe and conditions used for CPA I and II were also used to hybridize BD Bioscience's Cancer Cell Line Profiling Array, which contains cDNA from cell lines representing cancer of the lung (A549, NCI-H-460, NCI-H1299), colon (HCT-116, HCT-15, HT-29), breast (MDA-MB4355, MDA-MB231, MCF7), ovary (SK-OV-3), cervix (HeLa), prostate (DU-145, PC-3), skin (SK-MEL-28, SK-MEL-5, SK-NSH, IMR-32, U-87MG), brain (IMR-32, U-87MG), kidney (786-O, ACHN), liver (Hep-G2), colon (Colo589), osteosarcoma (U2-OS), and epidermis (A-431).

The results showed that the highest expression was observed in lung NCI-H1299 and A549 cells (Figure 4, left panel), kidney ACHN cells and U2-OS osteosarcoma cells (Figure 4, right panel). Lower but significant expression was observed in renal line, 786-O (Figure 4, right panel), MDA-MB-231 breast cells and ovarian cancer cell line, SKOV-3 (Figure 4, left panel).

### Example 6

The present example demonstrates S30-21616/DEGA cDNA cloning. Briefly, Human *S30-21616*/DEGA cDNA was amplified as a RT-PCR product derived from Marathon-Ready™ A549, a human lung cell carcinoma line and spleen cDNA preparations (BD Biosciences/Clonetech, Palo Alto, CA). The PCR primers used to amplify the polynucleotide of interest are forward PCR Primer No. 1 with the sequence 5' ATG TCG TTA CGT GTA CAC ACT CTG 3' (SEQ ID NO: 14) and reverse PCR Primer No. 2, 5'TTA AGT GGA CGC CAC AAA AGG TGT G 3' (SEQ ID NO:15) (Bio-Synthesis Incorporated, Lewisville, TX; start and stop codons are underlined). The PCR reaction was performed using standard RT-PCR protocols well known in the art, with the exception of the polymerase used, which is Titanium Taq polymerase (BD Biosciences/Clonetech, Palo Alto, CA), specifically the RT-PCR condition described in the Marathon-Ready^{™} cDNA manual was utilized: 94°C, 30 seconds; 68°C, 2 minutes.

The *S30-21616*/DEGA ORF PCR product (1569 bp) was cloned into pCR2.1 (InVitrogen Corporation, TOPO TA^{®} Cloning Kit, Carlsbad, CA) and sequence verified (Molecular Genetics Instrumentation Facility, University of Georgia, Athens, GA; GENEWIZ Inc, North Brunswick, NJ). To obtain DNA sequence upstream of the *S30-216161DEGA* start codon; 5' RACE was performed using A549 Marathon-Ready™ cDNA as explained above with few modifications. AP1, a forward primer (included with the Marathon-Ready™ cDNA) and a *S30-21616*/DEGA gene-specific reverse primer (5' AAC TCA GGT CCA GTC TCT TAA TCA G 3'; SEQ ID NO:16) produced a PCR product after 35 rounds of amplification (94°C, 30s; 57°C, 30 sec; 68°C, 2 min.) that was sub-cloned into pCR2.1 and sequenced verified. Sequence representing the 3'UTR of *S30-21616*/DEGA was initially obtained computationally from the human chromosome 12 BAC clone, GS1-99H8 (GenBank^{®} accession #AC004010). A series of PCR primers were subsequently designed to determine the longest 3' UTR sequence present in the A549 cDNA preparation. The following primer set was used to identify the longest 3' UTR segment: forward primer 5' ATG TCG TTA CGT GTA CAC ACT CTG 3' (SEQ ID NO: 17); start codon underlined and reverse primer 5'CAA AAT GAA AAG ACA GGC AAA CAA ATG 3' (SEQ ID NO:18), which initiates at X nucleotides 3' of the *S30-21616*/DEGA ORF.

Figure 5A schematically shows the structural features of the gene product of *S30-21616*/DEGA*.* The protein possesses a signal sequence and a transmembrane domain suggesting that it may localize to the cell surface. Its putative extracellular portion contains an Ig domain and 5 leucine-rich repeats (LRR) flanked by cysteine residues present in LRR-N-terminal (LRR-NT) and LRR-C-terminal (LRR-CT) domains. Consequently, *S30-21616*/DEGA appears to belong to the LRR superfamily. The putative cytosolic portion of *S30-21616*/DEGA contains 102 amino acids and lacks the presence of any known protein domains. Approximately 1/5 or 20 of the cytosolic residues are either a serine or a threonine, suggesting that *S30-27676*/DEGA may function as a signaling molecule in the cell. Taken together, protein sequence analysis suggests that the *S30-21616*/DEGA gene product may function as a signaling cell adhesion molecule. The corresponding amino acid sequence of the protein is illustrated in Figure 5B.

### Example 7

The present example demonstrates stable expression of an S30-21616/DEGA-EGFP fusion construct in 293 cells and sub-cellular localization of the protein. Briefly, a fusion protein was engineered whereby an enhanced green fluorescent protein (EGFP) was fused to its C-terminus. The S30-21616/DEGA ORF was PCR amplified and cloned into XhoI/AgeI sites of pEGFP-N1 (BD Biosciences Clontech, Palo Alto, CA). The PCR reaction was performed as described in the S30-21616/DEGA cDNA cloning section with the exception that the forward primer used (5' ATC CTC GAG GCG ACC ATA ATG TCG TTA CGT GTA CAC ACT 3'; SEQ ID NO:19), which contained the native Kozak sequence and a Xho I site (underlined) 5' to the start codon shown in bold. The reverse primer used (5'GAT CAC CGG TGC AGT GGA CGC CAC AAA AGG TGT GTC 3'; SEQ ID NO:20) contained an Age I site (underlined) 3' of the stop codon shown in bold.

The *S30-21616*/DEGA-EGFP construct was stably transfected into 293 cells using FuGENE6 transfection reagent (Roche Molecular Biochemicals, Indianapolis, IN) as outlined by the manufacturer. A 2:1 FuGENE6:DNA ratio was used. Transfected cells were left for 2 days at 37°C after which time they were plated in limiting dilution and subjected to selection with 800 µg/ml Geneticin (Sigma-Aldrich Corp., St. Louis, MO). Individual clones were isolated by trypsinization using cloning rings and allowed to expand.

To determine the sub-cellular localization of the *S30-21616*/DEGA-EGFP fusion protein, clones were assessed using a Zeiss Axioskop fluorescent microscope (200x magnification). The fluorescent micrograph shown in Figure 7 confirmed cell surface localization of the *S30-21616*/DEGA protein.

### Example 8

The present example demonstrates stable expression of S30-21616/DEGA antisenseconstructs in AGS cells. The expression profile of *S30-21616*/DEGA suggests that it may play a functional role in the development or progression of a subset of human gastric adenocarcinoma. To address the functional role of *S30-21616*/DEGA, human AGS cell line that has been shown to express significant levels of *S30-21616*/DEGA mRNA was transfected to stably express antisense*S30-21616*/DEGA construct or an empty vector. The cells lines established were used in comparison studies of clones in cell cycle, proliferation and tumorigenicity assays.

To generate *S30-21616*/DEGA antisenseclones, a nucleotide fragment encompassing the entire ORF of *S30-21616*/DEGA as well as one that comprised the 5' most 608 nucleotides of its ORF (generated from a partial Eco-RI digest of pCR2.1-*S30-21616*/DEGA) were cloned into pIRES PURO2 (BD Biosciences Clontech, Palo Alto, CA) in reverse orientation and stably transfected into AGS cells. Concurrently, pIRES PURO2 was also stably transfected into these cells to serve as an empty vector control. Transfection and clone isolation were performed exactly as described for the *S30-21616*/DEGA-EGFP fusion with the exception that cells were selected with 400 µg/ml puromycin (Sigma-Aldrich Corp., St. Louis, MO).

### Example 9

The present example demonstrates comparative expression of AGS/DEGA antisenseand empty vector clones by Northern Blot snalysis. To determine the effect of *S30-21616*/DEGA expression in gastric adenocarcinoma cell line, AGS stably transfected with antisense*S30-21616*/DEGA and empty vector, standard Northern-blotting procedures were carried out. Total RNA was isolated using the Micro-to-Midi Total RNA system (InVitrogen Corporation, Carlsbad, CA) exactly as recommended by the manufacturer and 5 µg was resolved on a 1.2% denaturing formaldehyde gel and transferred to Nytran^{®} SuPerCharge membranes (Schleicher & Schuell, Inc., Keene, NH). Blots were hybridized with the ³²P random-labeled *S30-21616*/DEGA C-terminal probe used to probe the CPA I and II cDNA blots. Hybridizations were performed for one hour at 68°C using ExpressHyb™ solution (BD Biosciences Clontech, Palo Alto, CA). Three 10 minute room temperature washes (2 X SSC, 0.05% SDS) and two 20 minute 50°C washes (0.1 X SSC, 0.1% SDS) followed. Membranes were rinsed in 2 X SSC and exposed overnight at -70°C to Kodak BioMax MR film. (Eastman Kodak Company, Rochester, NY)

Northern blot analysis of AGS/DEGA antisenseclones showed significant and stable down-regulation of *S30-21616*/DEGA expression. Figure 8 shows a decreased in level of mRNA expression for AGS/DEGA antisenseclone #6 (lane 3) and clone #11 (lane 4) compared to untransfected or wild type (WT) AGS parental cells (lane 1) and AGS empty vector clone #15 (lane 2). The lower panel shows the mRNA expression of β-actin in the cells tested as controls.

### Example 10

The present example demonstrates DNA content/cell cycle profiles of AGS/DEGA antisenseclones and empty vector clones by flow cytometry analysis. To measure DNA content and therefore cell cycle profile, flow cytometry was utilized. AGS empty vector and *S30-21616*/DEGA antisenseclones were grown asynchronously in serum-containing growth media, trypsinized, washed once in PBS and stained for 10 minutes at room temperature in the dark with the propidium iodide solution provided in the DNA QC Particles kit (Catalog # 349523; Becton Dickinson). Cells were subsequently analyzed on a Becton Dickinson FACSVantage™ flow cytometer using parameters outlined in the DNA QC particles kit. Cell size was assessed by light mircroscopy.

Flow cytometric analysis of the propidium iodide stained cells showed that AGS/empty vector cells demonstrated a typical DNA content/cell cycle phase profile for proliferating cells, with a majority of cells in Gₒ/G₁, followed in quantity by cells in G₂/M and S phase *(see,* Figure 9, left panel, top histogram). In contrast AGS/DEGA antisenseclones #6 (*see,* Figure 9, left panel, middle histogram) and #11 (*see,* Figure 9, left panel, bottom histogram) displayed a dramatic alteration in the cell cycle profile. Nearly all cells displayed a shift towards the G₂/M phase of the cell cycle. Clone #11 showed slightly greater shift towards the G₂/M phase than clone #6. The shift to G₂/M also correlated to the increase in cell size observed for both AGS/DEGA antisenseclone #6 and #11 *(see,* Figure 9, bottom two right panel) in contrast to the cell size observed for AGS/empty vector cells *(see,* Figure 9, top right panel).

### Example 11

The present example demonstrates in-vivo tumorigenicity studies of DEGA-expressing cells. Given the cell cycle profile differences observed between AGS/empty vector and AGS/DEGA antisenseclones, a difference in cell proliferation *in vivo* is also expected. Since AGS cells are tumorigenic in nude mice, the effect of *S30-21616*/DEGA antisensein suppressing *S30-21616*/DEGA expression and hence negatively influence the tumorigenic potential of AGS cells was carried out as follows. AGS clones were suspended in growth media, placed on ice and thoroughly mixed with MATRIGEL™ (Collaborative Research Biochemicals, Bedford, MA) at a 1:1 (v/v) ratio. MATRIGEL™ is derived from the Engelbroth-Holm-Swarm mouse sarcoma, which has been found to be a rich source of the ECM proteins: laminin, collagen IV, nidogen/enactin and proteoglycan (12). When mixed with cell lines, MATRIGEL™ enhances tumorigenesis (27). For each athymic (nu/nu) mouse (Charles River Laboratories, Wilmington, MA), ten million cells were injected subcutaneously and tumor volumes (mm³) were measured weekly using calipers for 11 weeks.

Figure 10 depicts the results of tumor growth study in mice. After 75 days, 12/12 AGS cell-injected mice and 19/19 AGS/empty vector clone #15 injected mice developed tumor with a mean tumor volume of approximately 800 mm³. In sharp contrast, only 6/12 mice injected with AGS/DEGA antisenseclone #6 and 6/19 mice injected with AGS/DEGA antisenseclone #11 developed tumors with a mean tumor volume of approximately 20 mm³.

### SEQ ID NO: 1

### hS30-21616/DEGA cDNA sequence:

The open reading frame is shown in upper case. Bold letters = start and stop codons. For more info on sequence, see Figure 1 of manuscript.

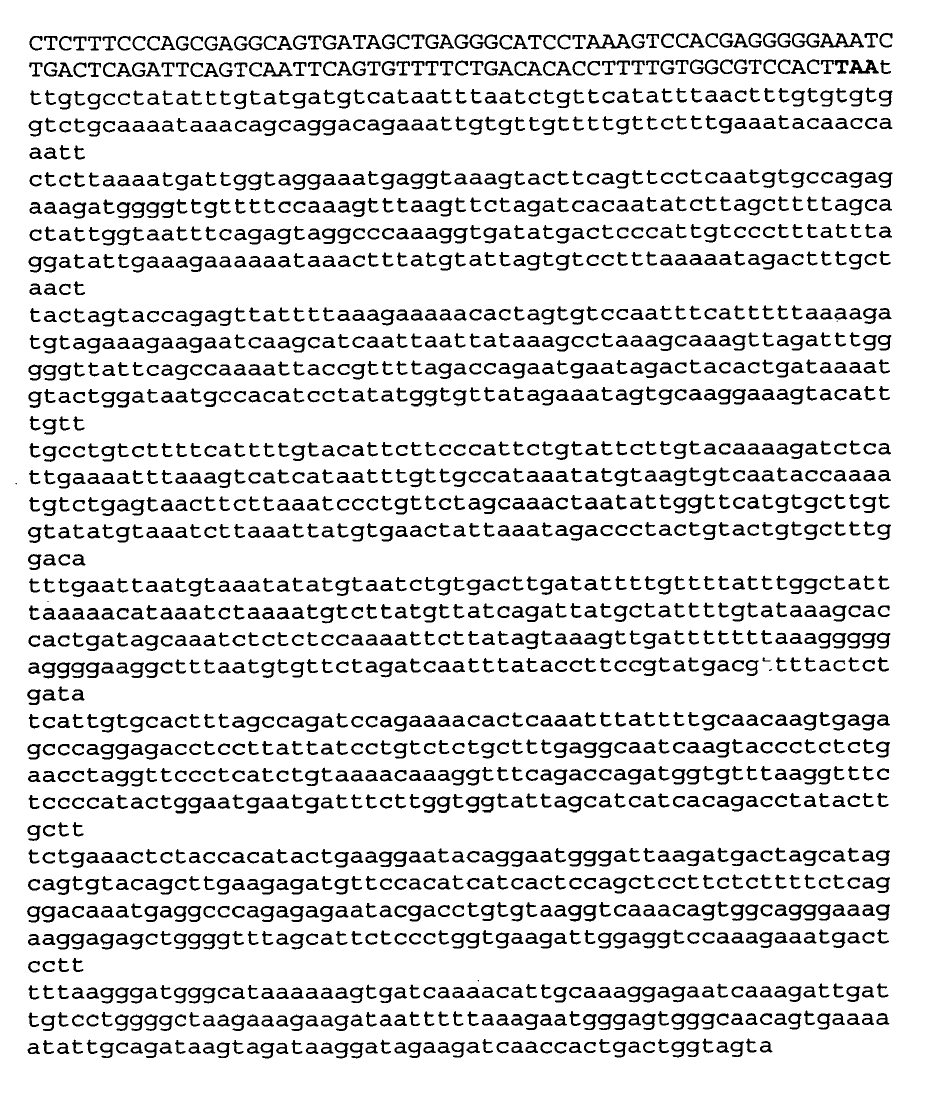

ggggtcctgcagcctcccgagtgcggagaggcggggccgcccgctgccgcccggctgcc
tgcgcccccctcccgcggccccggctctgggagcggggcgccccgcgcgcgggcacacgg
cggccagagcgccgaggcggtaccttcagcctgcaatgagaggaacccgggagagcccc
cgggagccagcgaagagcttggctgctgcgtccagggctgctgctgccgccgcggctgc
ttga
aactcctcaaagttgagagccggctagagggtgccgcccgccgggagccggagggaaag
gaagtcgggaggtgcaagagtgacagacacggacagacggacgcgcagaccttcggaag
gcactgcgtaggcagcctccccggagcccacgaggctccccagcaccgttcactggtgg
gaggctgagccggtggaaagacaccgggaagagactcagaggcgaccataATGTCGTT
ACGT
GTACACACTCTGCCCACCCTGCTTGGAGCCGTCGTCAGACCGGGCTGCAGGGAGCTGCT
GTGTTTGCTGATGATCACAGTGACTGTGGGCCCTGGTGCCTCTGGGGTGTGCCCCACCG
CTTGCATCTGTGCCACTGACATCGTCAGCTGCACCAACAAAAACCTGTCCAAGGTGCCT
GGGAACCTTTTCAGACTGATTAAGAGACTGGACCTGAGTTATAACAGAATTGGGCTTCT
GGAT
TCTGAGTGGATTCCAGTATCGTTTGCAAAGCTGAACACCCTAATTCTTCGTCATAACAA
CATCACCAGCATTTCCACGGGCAGTTTTTCCACAACTCCAAATTTGAAGTGTCTTGACT
TATCGTCCAATAAGCTGAAGACGGTGAAAAATGCTGTATTCCAAGAGTTGAAGGTTCTG
GAAGTGCTTCTGCTTTACAACAATCACATATCCTATCTCGATCCTTCAGCGTTTGGAGG
GCTC
TCCCAGTTGCAGAAACTCTACTTAAGTGGAAATTTTCTCACACAGTTTCCGATGGATTT
GTATGTTGGAAGGTTCAAGCTGGCAGAACTGATGTTTTTAGATGTTTCTTATAACCGAA
TTCCTTCCATGCCAATGCACCACATAAATTTAGTGCCAGGAAAACAGCTGAGAGGCATC
TACCTTCATGGAAACCCATTTGTCTGTGACTGTTCCCTGTACTCCTTGCTGGTCTTTTG
GTAT
CGTAGGCACTTTAGCTCAGTGATGGATTTTAAGAACGATTACACCTGTCGCCTGTGGTC
TGACTCCAGGCACTCGCGTCAGGTACTTCTGCTCCAGGATAGCTTTATGAATTGCTCTG
ACAGCATCATCAATGGTTCCTTTCGTGCGCTTGGCTTTATTCATGAGGCTCAGGTCGGG
GAAAGACTGATGGTCCACTGTGACAGCAAGACAGGTAATGCAAATACGGATTTCATCTG
GGTG
GGTCCAGATAACAGACTGCTAGAGCCGGATAAAGAGATGGAAAACTTTTACGTGTTTCA
CAATGGAAGTCTGGTTATAGAAAGCCCTCGTTTTGAGGATGCTGGAGTGTATTCTTGTA
TCGCAATGAATAAGCAACGCCTGTTAAATGAAACTGTGGACGTCACAATAAATGTGAGC
AATTTCACTGTAAGCAGATCCCATGCTCATGAGGCATTTAACACAGCTTTTACCACTCT
TGCT
GCTTGCGTGGCCAGTATCGTTTTGGTACTTTTGTACCTCTATCTGACTCCATGCCCCTG
CAAGTGTAAAACCAAGAGACAGAAAAATATGCTACACCAAAGCAATGCCCATTCATCGA
TTCTCAGTCCTGGCCCCGCTAGTGATGCCTCCGCTGATGAACGGAAGGCAGGTGCAGGT
AAAAGAGTGGTGTTTTTGGAACCCCTGAAGGATACTGCAGCAGGGCAGAACGGAAAGT
CAGG

### SEQ ID NO:2

### hS30-21616/DEGA amino acid sequence:

MSLRVHTLPTLLGAVVRPGCRELLCLLMITVTVGPGASGVCPTACICATDIVSCTNKNL
SKVPGNLFRLIKRLDLSYNRIGLLDSEWIPVSFAKLNTLILRHNNITSISTGSFSTTPN
LKCLDLSSNKLKTVKNAVFQELKVLEVLLLYNNHISYLDPSAFGGLSQLQKLYLSGNFL
TQFPMDLYVGRFKLAELMFLDVSYNRIPSMPMHHINLVPGKQLRGIYLHGNPFVCDCSL
YSLL
VFWYRRHFSSVMDFKNDYTCRLWSDSRHSRQVLLLQDSFMNCSDSIINGSFRALGFIHE
AQVGERLMVHCDSKTGNANTDFIWVGPDNRLLEPDKEMENFYVFHNGSLVIESPRFEDA
GVYSCIAMNKQRLLNETVDVTINVSNFTVSRSHAHEAFNTAFTTLAACVASIVLVLLYL
YLTPCPCKCKTKRQKNMLHQSNAHSSILSPGPASDASADERKAGAGKRVVFLEPLKDTA
AGQN
GKVRLFPSEAVIAEGILKSTRGKSDSDSVNSVFSDTPFVAST.

### SEQ ID NO: 3

### Polynucleotide sequence of mouse S30-21616, mS30-21616/DEGA.

ATGGTGAGCCCCAGCGCCTCAGGAATGTGCCCCACTGCTTGCATCTGTGCC
ACCGACATTGTCAGCTGCACCAACAAAAACCTATCTAAGGTGCCCGGAA
CCTTTTCAGACTGATTAAAAGACTGGATCTGAGCTATAACAGAATCGGAC
TGTTGGATGCCGACTGGATCCCGGTGTCGTTTGTCAAGCTGAGCACCTTAA
TTCTTCGCCACAACAACATCACCAGCATCTCCACGGCAGTTTCTCCACAA
CCCCAAATTTAAAGTGTCTGGACTTATCATCCAATAGGCTGAAGTCGGTA
AAGAGTGCCACATTCCAAGAGCTGAAGGCTCTGGAAGTACTGCTGCTGTA
CAACAACCACATTTCCTATCTGGACCCCGCAGCGTTCGGGGGGCTTTCCCA
CTTGCAGAAACTCTATCTGAGTGGGAACTTTCTCACACAGTTCCCTATGGA
TTTGTATACTGGGAGGTTCAAGCTGGCTGATCTGACATTTTTAGATGTTTC
CTATAATCGAATCCCTTCCATACCGATGCACCATATAAACTTAGTGCCGGG
GAGACAGCTGAGAGGCATCTACCTTCACGGAACCCATTTGTATGTGACT
GTTCTCTGTACTCGTTGCTGATCTTTTGGTACCGTAGGCACTTTAGCTCCGT
GATGGATTTTAAGAATGACTATACCTGTCGCCTGTGGTCTGACTCCAGGCA
CTCCCACCAGCTGCAGCTGCTCCAGGAGAGCTTTCTGAACTGTTCTTACAG
CGTTATCAACGGCTCCTTCCACGCACTTGGCTTTATCCACGAGGCTCAGGT
TGGGGAGAGGGCGATCGTCCACTGTGACAGCAAGACTGGCAATGGAAAT
ACTGATTTCATCTGGGTCGGTCCCGATAACAGGCTGCTGGAGCCAGATAA
AGACATGGGGAACTTTCGTGTGTTTACAACGGAAGTCTGGTCATAGAGA
ACCCTGGCTTTGAGGACGCCGGGGTATATTCTTGTATCGCAATGAACAGG
CAGCGGCTGTTAA[?]CGAGACGGTGGATATCATGATCAACGTGAGCAATTT
CACCATAAACAGATCCCACGCCCACGAGGCGTTTAACACGGCCTTTACCA
CCCTGGCTGCCTGCGTGGCCAGTATAGTT
CTAGTGCTACTGTATCTGTACCTGACGCCGTGCCCATGCAAATGCAAAGCC
AAGAGACAGAAAAACACGCTGAGCCAAAGCAGTGCCCACTCGTCCATTCT
CAGTCCTGGCCCCACTGGCGATGCCTCTGCTGACGATCGGAAGGCAGGTA
AAAGAGTCGTGTTTCTGGAGCCCCTGAAGGACACGGCGGCCGGACAGAAT
GGCAAAGTCAAGCTTTTCCCCAGTGAGACCGTTATAGCCGAGGGCATCTT
AAAGTCCACCAGGGCAAAGTCTGACTCAGACTCAGTCAATTCCGTGTTCT
CAGACACACCCTTTGTGGCATCCACTTAG

### SEQ ID NO: 4

### Amino acid sequence of mouse S30-21616 polypeptide, mS30-21616/DEGA.

MVSPSASGMCPTACICATDIVSCTNKNLSKVPGNLFRLIKRLDLSYNRIGLLDA
DWIPVSFVKLSTLILRHNNITSISTGSFSTTPNLKCLDLSSNRLKSVKSATFQEL
KALEVLLLYNNHISYLDPAAFGGLSHLQKLYLSGNFLTQFPMDLYTGRFKLA
DLTFLDVSYNRIPSIPMHHINLVPGRQLRGIYLHGNPFVCDCSLYSLLIFWYRR
HFSSVMDFKNDYTCRLWSDSRHSHQLQLLQESFLNCSYSVINGSFHALGFIHE
AQVGERAIVHCDSKTGNGNTDFIWVGPDNRLLEPDKDMGNFRVFYNGSLVIE
NPGFEDAGVYSCIAMNRQRLLNETVDIMINVSNFTINRSHAHEAFNTAFTTLA
ACVASIVLVLLYLYLTPCPCKCKAKRQKNTLSQSSAHSSILSPGPTGDASADD
RKAGKRVVFLEPLKDTAAGQNGKVKLFPSETVIAEGILKSTRAKSDSDSVNSV
FSDTPFVAST

### SEQ ID NO: 5

### Amino acid sequence of human S30-21616/DEGA polypeptide lacking transmembrane (TM) domain, -TM hS30-21616/DEGA.

MSLRVHTLPTLLGAVVRPGCRELLCLLMITVTVGPGASGVCPTACICATDIVS
CTNKNLSKVPGNLFRLIKRLDLSYNRIGLLDSEWIPVSFAKLNTLILRHNNITSI
STGSFSTTPNLKCLDLSSNKLKTVKNAVFQELKVLEVLLLYNNHISYLDPSAF
GGLSQLQKLYLSGNFLTQFPMDLYVGRFKLAELMFLDVSYNRIPSMPMHHIN
LVPGKQLRGIYLHGNPFVCDCSLYSLLVFWYRRHFSSVMDFKNDYTCRLWS
DSRHSRQVLLLQDSFMNCSDSIINGSFRALGFIHEAQVGERLMVHCDSKTGNA
NTDFIWVGPDNRLLEPDKEMENFYVFHNGSLVIESPRFEDAGVYSCIAMNKQ
RLLNETVDVTINVSNFTVSRSHAHEAFNT

### SEQ ID NO:6 Leucine Zipper Motif

LYLSGNFLTQFPMDLYVGRFKL

### SEQ ID NO: 7 Leucine Rich Repeat 1

VCPTACICATDIVSCTNKNLSKVPGNLFRLIKR

### SEQ ID NO: 8 Leucine Rich Repeat 2

SFAKLNTLILRHNNI

### SEQ ID NO: 9 Leucine Rich Repeat 3

TSISTGSFSTTPNLKCLDLSSNKLKTVKNAVFQELKVLEVLLLYN

### SEQ ID NO: 10 Leucine Rich Repeat 4

LKVLEVLLLYNNHISYLDPSAFGG

### SEQ ID NO: 11 Leucine Rich Repeat 5

LSQLQKLYLSGNFLTQFPMDLYVG

### SEQ ID NO: 12 Leucine Rich Repeat 6

LAELMFLDVSYNRIPSMPMHHINLV

### SEQ ID NO: 13 Immunoglobulin (Ig)- domain

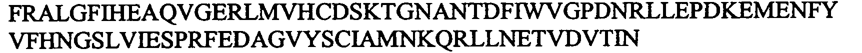

### SEQ ID NO: 14 PCR Primer 1

5'-ATG TCG TTA CGT GTA CAC ACT CTG - 3'

### SEQ ID NO: 15 PCR Primer 2

5'-TTA AGT GGA CGC CAC AAA AGG TGT- 3'

### SEQ ID NO:16 S30-21616/DEGA Gene -specific Reverse Primer

5'-AAC TCA GGT CCA GTC TCT TAA TCA G- 3'

### SEQ ID NO:17 Forward primer

5'- ATG TCG TTA CGT GTA CAC ACT CTG-3'

### SEQ ID NO:18 Reverse Primer

5'- CAA AAT GAA AAG ACA GGC AAA CAA ATG-3'

### SEQ ID NO:19 Forward Primer for S30-21616/DEGA-EGFP Fusion Construct

5'ATC CTC GAG GCG ACC ATA ATG TCG TTA CGT GTA CAC ACT 3'

### SEQ ID NO:20 Reverse Primer for S30-21616/DEGA-EGFP Fusion Construct

5' GAT CAC CGG TGC **AGT** GGA CGC CAC AAA AGG TGT GTC 3'

## Claims

1. An antibody or fragment thereof, that binds to a polypeptide of SEQ ID No. 2 or SEQ ID No. 4 for use in the treatment of cancer.

2. The antibody of fragment thereof for use according to claim 1, wherein the cancer is gastric adenocarcinoma.

3. The antibody or fragment thereof for use according to claim 1 or claim 2, wherein the antibody or fragment is conjugated to a radioisotope, a cytotoxic or a chemotherapeutic agent.

4. The antibody or fragment thereof for use according to any one of claims 1 to 3, wherein said antibody is human or humanized.

5. An antibody or fragment thereof, that binds to a polypeptide of SEQ ID No. 2 or SEQ ID No. 4 for use in the diagnosis of cancer.

6. The antibody or fragment thereof, for use according to claim 5, wherein the cancer is gastric adenocarcinoma.


SEQUENCE LISTING

<110> IMCLONE SYSTEMS INCORPORATED

<120> NOVEL POLYNUCLEOTIDE AND POLYPEPTIDE SEQUENCES AND USES
      THEREOF

<130> 11245/49076

<140> PCT/US03/20601
<141> 2003-06-30

<150> 60/392,675
<151> 2002-06-28

<160> 20

<170> PatentIn Ver. 2.1

<210> 1
<211> 3769
<212> DNA
<213> Homo sapiens

<220>
<221> CDS
<222> (469)..(2034)

<400> 1
   ggggtcctgc agcctcccga gtgcggagag gcggggccgc ccgctgccgc ccggctgcct 60
   gcgccccctc ccgcggcccc ggctctggga gcggggcgcc ccgcgcgcgg gcacacggcg 120
   gccagagcgc cgaggcggta ccttcagcct gcaatgagag gaacccggga gagcccccgg 180
   gagccagcga agagcttggc tgctgcgtcc agggctgctg ctgccgccgc ggctgcttga 240
   aactcctcaa agttgagagc cggctagagg gtgccgcccg ccgggagccg gagggaaagg 300
   aagtcgggag gtgcaagagt gacagacacg gacagacgga cgcgcagacc ttcggaaggc 360
   actgcgtagg cagcctcccc ggagcccacg aggctcccca gcaccgttca ctggtgggag 420
   gctgagccgg tggaaaagac accgggaaga gactcagagg cgaccata atg tcg tta 477
                                                        Met Ser Leu
                                                          1
   cgt gta cac act ctg ccc acc ctg ctt gga gcc gtc gtc aga ccg ggc 525
   Arg Val His Thr Leu Pro Thr Leu Leu Gly Ala Val Val Arg Pro Gly
         5                  10                  15
   tgc agg gag ctg ctg tgt ttg ctg atg atc aca gtg act gtg ggc cct 573
   Cys Arg Glu Leu Leu Cys Leu Leu Met Ile Thr Val Thr Val Gly Pro
    20                  25                  30                  35
   ggt gcc tct ggg gtg tgc ccc acc gct tgc atc tgt gcc act gac atc 621
   Gly Ala Ser Gly Val Cys Pro Thr Ala Cys Ile Cys Ala Thr Asp Ile
                    40                  45                  50
   gtc agc tgc acc aac aaa aac ctg tcc aag gtg cct ggg aac ctt ttc 669
   Val Ser Cys Thr Asn Lys Asn Leu Ser Lys Val Pro Gly Asn Leu Phe
                55                  60                  65
   aga ctg att aag aga ctg gac ctg agt tat aac aga att ggg ctt ctg 717
   Arg Leu Ile Lys Arg Leu Asp Leu Ser Tyr Asn Arg Ile Gly Leu Leu
            70                  75                  80
   gat tct gag tgg att cca gta tcg ttt gca aag ctg aac acc cta att 765
   Asp Ser Glu Trp Ile Pro Val Ser Phe Ala Lys Leu Asn Thr Leu Ile
        85                  90                  95
   ctt cgt cat aac aac atc acc agc att tcc acg ggc agt ttt tcc aca 813
   Leu Arg His Asn Asn Ile Thr Ser Ile Ser Thr Gly Ser Phe Ser Thr
   100                 105                 110                 115
   act cca aat ttg aag tgt ctt gac tta tcg tcc aat aag ctg aag acg 861
   Thr Pro Asn Leu Lys Cys Leu Asp Leu Ser Ser Asn Lys Leu Lys Thr
   120 125 130
   gtg aaa aat gct gta ttc caa gag ttg aag gtt ctg gaa gtg ctt ctg 909
   Val Lys Asn Ala Val Phe Gln Glu Leu Lys Val Leu Glu Val Leu Leu
   135 140 145
   ctt tac aac aat cac ata tcc tat ctc gat cct tca gcg ttt gga ggg 957
   Leu Tyr Asn Asn His Ile Ser Tyr Leu Asp Pro Ser Ala Phe Gly Gly
   150 155 160
   ctc tcc cag ttg cag aaa ctc tac tta agt gga aat ttt ctc aca cag 1005
   Leu Ser Gln Leu Gln Lys Leu Tyr Leu Ser Gly Asn Phe Leu Thr Gln
   165 170 175
   ttt ccg atg gat ttg tat gtt gga agg ttc aag ctg gca gaa ctg atg 1053
   Phe Pro Met Asp Leu Tyr Val Gly Arg Phe Lys Leu Ala Glu Leu Met
   180 185 190 195
   ttt tta gat gtt tct tat aac cga att cct tcc atg cca atg cac cac 1101
   Phe Leu Asp Val Ser Tyr Asn Arg Ile Pro Ser Met Pro Met His His
   200 205 210
   ata aat tta gtg cca gga aaa cag ctg aga ggc atc tac ctt cat gga 1149
   Ile Asn Leu Val Pro Gly Lys Gln Leu Arg Gly Ile Tyr Leu His Gly
   215 220 225
   aac cca ttt gtc tgt gac tgt tcc ctg tac tcc ttg ctg gtc ttt tgg 1197
   Asn Pro Phe Val Cys Asp Cys Ser Leu Tyr Ser Leu Leu Val Phe Trp
   230 235 240
   tat cgt agg cac ttt agc tca gtg atg gat ttt aag aac gat tac acc 1245
   Tyr Arg Arg His Phe Ser Ser Val Met Asp Phe Lys Asn Asp Tyr Thr
   245 250 255
   tgt cgc ctg tgg tct gac tcc agg cac tcg cgt cag gta ctt ctg ctc 1293
   Cys Arg Leu Trp Ser Asp Ser Arg His Ser Arg Gln Val Leu Leu Leu
   260 265 270 275
   cag gat agc ttt atg aat tgc tct gac agc atc atc aat ggt tcc ttt 1341
   Gln Asp Ser Phe Met Asn Cys Ser Asp Ser Ile Ile Asn Gly Ser Phe
   280 285 290
   cgt gcg ctt ggc ttt att cat gag gct cag gtc ggg gaa aga ctg atg 1389
   Arg Ala Leu Gly Phe Ile His Glu Ala Gln Val Gly Glu Arg Leu Met
   295 300 305
   gtc cac tgt gac agc aag aca ggt aat gca aat acg gat ttc atc tgg 1437
   Val His Cys Asp Ser Lys Thr Gly Asn Ala Asn Thr Asp Phe Ile Trp
   310 315 320
   gtg ggt cca gat aac aga ctg cta gag ccg gat aaa gag atg gaa aac 1485
   Val Gly Pro Asp Asn Arg Leu Leu Glu Pro Asp Lys Glu Met Glu Asn
   325 330 335
   ttt tac gtg ttt cac aat gga agt ctg gtt ata gaa agc cct cgt ttt 1533
   Phe Tyr Val Phe His Asn Gly Ser Leu Val Ile Glu Ser Pro Arg Phe
   340 345 350 355
   gag gat gct gga gtg tat tct tgt atc gca atg aat aag caa cgc ctg 1581
   Glu Asp Ala Gly Val Tyr Ser Cys Ile Ala Met Asn Lys Gln Arg Leu
   360 365 370
   tta aat gaa act gtg gac gtc aca ata aat gtg agc aat ttc act gta 1629
   Leu Asn Glu Thr Val Asp Val Thr Ile Asn Val Ser Asn Phe Thr Val
   375 380 385
   agc aga tcc cat gct cat gag gca ttt aac aca gct ttt acc act ctt 1677
   Ser Arg Ser His Ala His Glu Ala Phe Asn Thr Ala Phe Thr Thr Leu
   390 395 400
   gct gct tgc gtg gcc agt atc gtt ttg gta ctt ttg tac ctc tat ctg 1725
   Ala Ala Cys Val Ala Ser Ile Val Leu Val Leu Leu Tyr Leu Tyr Leu
   405 410 415
   act cca tgc ccc tgc aag tgt aaa acc aag aga cag aaa aat atg cta 1773
   Thr Pro Cys Pro Cys Lys Cys Lys Thr Lys Arg Gln Lys Asn Met Leu
   420 425 430 435
   cac caa agc aat gcc cat tca tcg att ctc agt cct ggc ccc gct agt 1821
   His Gln Ser Asn Ala His Ser Ser Ile Leu Ser Pro Gly Pro Ala Ser
   440 445 450
   gat gcc tcc gct gat gaa cgg aag gca ggt gca ggt aaa aga gtg gtg 1869
   Asp Ala Ser Ala Asp Glu Arg Lys Ala Gly Ala Gly Lys Arg Val Val
   455 460 465
   ttt ttg gaa ccc ctg aag gat act gca gca ggg cag aac ggg aaa gtc 1917
   Phe Leu Glu Pro Leu Lys Asp Thr Ala Ala Gly Gln Asn Gly Lys Val
   470 475 480
   agg ctc ttt ccc agc gag gca gtg ata gct gag ggc atc cta aag tcc 1965
   Arg Leu Phe Pro Ser Glu Ala Val Ile Ala Glu Gly Ile Leu Lys Ser
   485 490 495
   acg agg ggg aaa tct gac tca gat tca gtc aat tca gtg ttt tct gac 2013
   Thr Arg Gly Lys Ser Asp Ser Asp Ser Val Asn Ser Val Phe Ser Asp
   500 505 510 515
   aca cct ttt gtg gcg tcc act taatttgtgc ctatatttgt atgatgtcat 2064
   Thr Pro Phe Val Ala Ser Thr
   520
   aatttaatct gttcatattt aactttgtgt gtggtctgca aaataaacag caggacagaa 2124
   attgtgttgt tttgttcttt gaaatacaac caaattctct taaaatgatt ggtaggaaat 2184
   gaggtaaagt acttcagttc ctcaatgtgc cagagaaaga tggggttgtt ttccaaagtt 2244
   taagttctag atcacaatat cttagctttt agcactattg gtaatttcag agtaggccca 2304
   aaggtgatat gactcccatt gtccctttat ttaggatatt gaaagaaaaa ataaacttta 2364
   tgtattagtg tcctttaaaa atagactttg ctaacttact agtaccagag ttattttaaa 2424
   gaaaaacact agtgtccaat ttcattttta aaagatgtag aaagaagaat caagcatcaa 2484
   ttaattataa agcctaaagc aaagttagat ttgggggtta ttcagccaaa attaccgttt 2544
   tagaccagaa tgaatagact acactgataa aatgtactgg ataatgccac atcctatatg 2604
   gtgttataga aatagtgcaa ggaaagtaca tttgtttgcc tgtcttttca ttttgtacat 2664
   tcttcccatt ctgtattctt gtacaaaaga tctcattgaa aatttaaagt catcataatt 2724
   tgttgccata aatatgtaag tgtcaatacc aaaatgtctg agtaacttct taaatccctg 2784
   ttctagcaaa ctaatattgg ttcatgtgct tgtgtatatg taaatcttaa attatgtgaa 2844
   ctattaaata gaccctactg tactgtgctt tggacatttg aattaatgta aatatatgta 2904
   atctgtgact tgatattttg ttttatttgg ctatttaaaa acataaatct aaaatgtctt 2964
   atgttatcag attatgctat tttgtataaa gcaccactga tagcaaatct ctctccaaaa 3024
   ttcttatagt aaagttgatt tttttaaagg gggaggggaa ggctttaatg tgttctagat 3084
   caatttatac cttccgtatg acgttttact ctgatatcat tgtgcacttt agccagatcc 3144
   agaaaacact caaatttatt ttgcaacaag tgagagccca ggagacctcc ttattatcct 3204
   gtctctgctt tgaggcaatc aagtaccctc tctgaaccta ggttccctca tctgtaaaac 3264
   aaaggtttca gaccagatgg tgtttaaggt ttctccccat actggaatga atgatttctt 3324
   ggtggtatta gcatcatcac agacctatac ttgctttctg aaactctacc acatactgaa 3384
   ggaatacagg aatgggatta agatgactag catagcagtg tacagcttga agagatgttc 3444
   cacatcatca ctccagctcc ttctcttttc tcagggacaa atgaggccca gagagaatac 3504
   gacctgtgta aggtcaaaca gtggcaggga aagaaggaga gctggggttt agcattctcc 3564
   ctggtgaaga ttggaggtcc aaagaaatga ctccttttta agggatgggc ataaaaaagt 3624
   gatcaaaaca ttgcaaagga gaatcaaaga ttgattgtcc tggggctaag aaagaagata 3684
   atttttaaag aatgggagtg ggcaacagtg aaaaatattg cagataagta gataaggata 3744
   gaagatcaac cactgactgg tagta 3769

<210> 2
<211> 522
<212> PRT
<213> Homo sapiens

<400> 2
Met Ser Leu Arg Val His Thr Leu Pro Thr Leu Leu Gly Ala Val Val
1               5                   10                  15

Arg Pro Gly Cys Arg Glu Leu Leu Cys Leu Leu Met Ile Thr Val Thr
            20                  25                  30

Val Gly Pro Gly Ala Ser Gly Val Cys Pro Thr Ala Cys Ile Cys Ala
        35                  40                  45

Thr Asp Ile Val Ser Cys Thr Asn Lys Asn Leu Ser Lys Val Pro Gly
    50                  55                  60

Asn Leu Phe Arg Leu Ile Lys Arg Leu Asp Leu Ser Tyr Asn Arg Ile
65                  70                  75                  80

Gly Leu Leu Asp Ser Glu Trp Ile Pro Val Ser Phe Ala Lys Leu Asn
                85                  90                  95

Thr Leu Ile Leu Arg His Asn Asn Ile Thr Ser Ile Ser Thr Gly Ser
            100                 105                 110

Phe Ser Thr Thr Pro Asn Leu Lys Cys Leu Asp Leu Ser Ser Asn Lys
        115                 120                 125

Leu Lys Thr Val Lys Asn Ala Val Phe Gln Glu Leu Lys Val Leu Glu
    130                 135                 140

Val Leu Leu Leu Tyr Asn Asn His Ile Ser Tyr Leu Asp Pro Ser Ala
145                 150                 155                 160

Phe Gly Gly Leu Ser Gln Leu Gln Lys Leu Tyr Leu Ser Gly Asn Phe
                165                 170                 175

Leu Thr Gln Phe Pro Met Asp Leu Tyr Val Gly Arg Phe Lys Leu Ala
            180                 185                 190

Glu Leu Met Phe Leu Asp Val Ser Tyr Asn Arg Ile Pro Ser Met Pro
        195                 200                 205

Met His His Ile Asn Leu Val Pro Gly Lys Gln Leu Arg Gly Ile Tyr
    210                 215                 220

Leu His Gly Asn Pro Phe Val Cys Asp Cys Ser Leu Tyr Ser Leu Leu
225                 230                 235                 240

Val Phe Trp Tyr Arg Arg His Phe Ser Ser Val Met Asp Phe Lys Asn
                245                 250                 255

Asp Tyr Thr Cys Arg Leu Trp Ser Asp Ser Arg His Ser Arg Gln Val
            260                 265                 270

Leu Leu Leu Gln Asp Ser Phe Met Asn Cys Ser Asp Ser Ile Ile Asn
        275                 280                 285

Gly Ser Phe Arg Ala Leu Gly Phe Ile His Glu Ala Gln Val Gly Glu
    290                 295                 300

Arg Leu Met Val His Cys Asp Ser Lys Thr Gly Asn Ala Asn Thr Asp
305                 310                 315                 320

Phe Ile Trp Val Gly Pro Asp Asn Arg Leu Leu Glu Pro Asp Lys Glu
                325                 330                 335

Met Glu Asn Phe Tyr Val Phe His Asn Gly Ser Leu Val Ile Glu Ser

340                 345                 350

Pro Arg Phe Glu Asp Ala Gly Val Tyr Ser Cys Ile Ala Met Asn Lys
        355                 360                 365

Gln Arg Leu Leu Asn Glu Thr Val Asp Val Thr Ile Asn Val Ser Asn
    370                 375                 380

Phe Thr Val Ser Arg Ser His Ala His Glu Ala Phe Asn Thr Ala Phe
385                 390                 395                 400

Thr Thr Leu Ala Ala Cys Val Ala Ser Ile Val Leu Val Leu Leu Tyr
                405                 410                 415

Leu Tyr Leu Thr Pro Cys Pro Cys Lys Cys Lys Thr Lys Arg Gln Lys
            420                 425                 430

Asn Met Leu His Gln Ser Asn Ala His Ser Ser Ile Leu Ser Pro Gly
        435                 440                 445

Pro Ala Ser Asp Ala Ser Ala Asp Glu Arg Lys Ala Gly Ala Gly Lys
    450                 455                 460

Arg Val Val Phe Leu Glu Pro Leu Lys Asp Thr Ala Ala Gly Gln Asn
465                 470                 475                 480

Gly Lys Val Arg Leu Phe Pro Ser Glu Ala Val Ile Ala Glu Gly Ile
                485                 490                 495

Leu Lys Ser Thr Arg Gly Lys Ser Asp Ser Asp Ser Val Asn Ser Val
            500                 505                 510

Phe Ser Asp Thr Pro Phe Val Ala Ser Thr
        515                 520

<210> 3
<211> 1470
<212> DNA
<213> Mus musculus

<220>
<221> CDS
<222> (1)..(1470)

<400> 3
atg gtg agc ccc agc gcc tca gga atg tgc ccc act gct tgc atc tgt      48
Met Val Ser Pro Ser Ala Ser Gly Met Cys Pro Thr Ala Cys Ile Cys
1               5                   10                  15

gcc acc gac att gtc agc tgc acc aac aaa aac cta tct aag gtg ccc      96
Ala Thr Asp Ile Val Ser Cys Thr Asn Lys Asn Leu Ser Lys Val Pro
            20                  25                  30

ggg aac ctt ttc aga ctg att aaa aga ctg gat ctg agc tat aac aga      144
Gly Asn Leu Phe Arg Leu Ile Lys Arg Leu Asp Leu Ser Tyr Asn Arg
        35                  40                  45

atc gga ctg ttg gat gcc gac tgg atc ccg gtg tcg ttt gtc aag ctg      192
Ile Gly Leu Leu Asp Ala Asp Trp Ile Pro Val Ser Phe Val Lys Leu
    50                  55                  60

agc acc tta att ctt cgc cac aac aac atc acc agc atc tcc acg ggc      240
Ser Thr Leu Ile Leu Arg His Asn Asn Ile Thr Ser Ile Ser Thr Gly
65                  70                  75                  80

agt ttc tcc aca acc cca aat tta aag tgt ctg gac tta tca tcc aat      288
Ser Phe Ser Thr Thr Pro Asn Leu Lys Cys Leu Asp Leu Ser Ser Asn
                85                  90                  95

agg ctg aag tcg gta aag agt gcc aca ttc caa gag ctg aag gct ctg      336
Arg Leu Lys Ser Val Lys Ser Ala Thr Phe Gln Glu Leu Lys Ala Leu
            100                 105                 110

gaa gta ctg ctg ctg tac aac aac cac att tcc tat ctg gac ccc gca      384
Glu Val Leu Leu Leu Tyr Asn Asn His Ile Ser Tyr Leu Asp Pro Ala

        115                 120                 125

gcg ttc ggg ggg ctt tcc cac ttg cag aaa ctc tat ctg agt ggg aac 432
Ala Phe Gly Gly Leu Ser His Leu Gln Lys Leu Tyr Leu Ser Gly Asn
    130                 135                 140

ttt ctc aca cag ttc cct atg gat ttg tat act ggg agg ttc aag ctg 480
Phe Leu Thr Gln Phe Pro Met Asp Leu Tyr Thr Gly Arg Phe Lys Leu
145                 150                 155                 160

gct gat ctg aca ttt tta gat gtt tcc tat aat cga atc cct tcc ata 528
Ala Asp Leu Thr Phe Leu Asp Val Ser Tyr Asn Arg Ile Pro Ser Ile
                165                 170                 175

ccg atg cac cat ata aac tta gtg ccg ggg aga cag ctg aga ggc atc 576
Pro Met His His Ile Asn Leu Val Pro Gly Arg Gln Leu Arg Gly Ile
            180                 185                 190

tac ctt cac ggg aac cca ttt gta tgt gac tgt tct ctg tac tcg ttg 624
Tyr Leu His Gly Asn Pro Phe Val Cys Asp Cys Ser Leu Tyr Ser Leu
        195                 200                 205

ctg atc ttt tgg tac cgt agg cac ttt agc tcc gtg atg gat ttt aag 672
Leu Ile Phe Trp Tyr Arg Arg His Phe Ser Ser Val Met Asp Phe Lys
    210                 215                 220

aat gac tat acc tgt cgc ctg tgg tct gac tcc agg cac tcc cac cag 720
Asn Asp Tyr Thr Cys Arg Leu Trp Ser Asp Ser Arg His Ser His Gln
225                 230                 235                 240

ctg cag ctg ctc cag gag agc ttt ctg aac tgt tct tac agc gtt atc 768
Leu Gln Leu Leu Gln Glu Ser Phe Leu Asn Cys Ser Tyr Ser Val Ile
                245                 250                 255

aac ggc tcc ttc cac gca ctt ggc ttt atc cac gag gct cag gtt ggg 816
Asn Gly Ser Phe His Ala Leu Gly Phe Ile His Glu Ala Gln Val Gly
            260                 265                 270

gag agg gcg atc gtc cac tgt gac agc aag act ggc aat gga aat act 864
Glu Arg Ala Ile Val His Cys Asp Ser Lys Thr Gly Asn Gly Asn Thr
        275                 280                 285

gat ttc atc tgg gtc ggt ccc gat aac agg ctg ctg gag cca gat aaa 912
Asp Phe Ile Trp Val Gly Pro Asp Asn Arg Leu Leu Glu Pro Asp Lys
    290                 295                 300

gac atg ggg aac ttt cgt gtg ttt tac aac gga agt ctg gtc ata gag 960
Asp Met Gly Asn Phe Arg Val Phe Tyr Asn Gly Ser Leu Val Ile Glu
305                 310                 315                 320

aac cct ggc ttt gag gac gcc ggg gta tat tct tgt atc gca atg aac 1008
Asn Pro Gly Phe Glu Asp Ala Gly Val Tyr Ser Cys Ile Ala Met Asn
                325                 330                 335

agg cag cgg ctg tta aac gag acg gtg gat atc atg atc aac gtg agc 1056
Arg Gln Arg Leu Leu Asn Glu Thr Val Asp Ile Met Ile Asn Val Ser
            340                 345                 350

aat ttc acc ata aac aga tcc cac gcc cac gag gcg ttt aac acg gcc 1104
Asn Phe Thr Ile Asn Arg Ser His Ala His Glu Ala Phe Asn Thr Ala
        355                 360                 365

ttt acc acc ctg gct gcc tgc gtg gcc agt ata gtt cta gtg cta ctg 1152
Phe Thr Thr Leu Ala Ala Cys Val Ala Ser Ile Val Leu Val Leu Leu
    370                 375                 380

tat ctg tac ctg acg ccg tgc cca tgc aaa tgc aaa gcc aag aga cag 1200
Tyr Leu Tyr Leu Thr Pro Cys Pro Cys Lys Cys Lys Ala Lys Arg Gln
385                 390                 395                 400

aaa aac acg ctg agc caa agc agt gcc cac tcg tcc att ctc agt cct 1248
Lys Asn Thr Leu Ser Gln Ser Ser Ala His Ser Ser Ile Leu Ser Pro
                405                 410                 415

ggc ccc act ggc gat gcc tct gct gac gat cgg aag gca ggt aaa aga 1296
Gly Pro Thr Gly Asp Ala Ser Ala Asp Asp Arg Lys Ala Gly Lys Arg
            420                 425                 430

gtc gtg ttt ctg gag ccc ctg aag gac acg gcg gcc gga cag aat ggc     1344
Val Val Phe Leu Glu Pro Leu Lys Asp Thr Ala Ala Gly Gln Asn Gly
        435                 440                 445

aaa gtc aag ctt ttc ccc agt gag acc gtt ata gcc gag ggc atc tta     1392
Lys Val Lys Leu Phe Pro Ser Glu Thr Val Ile Ala Glu Gly Ile Leu
    450                 455                 460

aag tcc acc agg gca aag tct gac tca gac tca gtc aat tcc gtg ttc     1440
Lys Ser Thr Arg Ala Lys Ser Asp Ser Asp Ser Val Asn Ser Val Phe
465                 470                 475                 480

tca gac aca ccc ttt gtg gca tcc act tag                             1470
Ser Asp Thr Pro Phe Val Ala Ser Thr
                485


<210> 4
<211> 489
<212> PRT
<213> Mus musculus

<400> 4
Met Val Ser Pro Ser Ala Ser Gly Met Cys Pro Thr Ala Cys Ile Cys
  1               5                  10                  15

Ala Thr Asp Ile Val Ser Cys Thr Asn Lys Asn Leu Ser Lys Val Pro
             20                  25                  30

Gly Asn Leu Phe Arg Leu Ile Lys Arg Leu Asp Leu Ser Tyr Asn Arg
         35                  40                  45

Ile Gly Leu Leu Asp Ala Asp Trp Ile Pro Val Ser Phe Val Lys Leu
     50                  55                  60

Ser Thr Leu Ile Leu Arg His Asn Asn Ile Thr Ser Ile Ser Thr Gly
 65                  70                  75                  80

Ser Phe Ser Thr Thr Pro Asn Leu Lys Cys Leu Asp Leu Ser Ser Asn
                 85                  90                  95

Arg Leu Lys Ser Val Lys Ser Ala Thr Phe Gln Glu Leu Lys Ala Leu
            100                 105                 110

Glu Val Leu Leu Leu Tyr Asn Asn His Ile Ser Tyr Leu Asp Pro Ala
        115                 120                 125

Ala Phe Gly Gly Leu Ser His Leu Gln Lys Leu Tyr Leu Ser Gly Asn
    130                 135                 140

Phe Leu Thr Gln Phe Pro Met Asp Leu Tyr Thr Gly Arg Phe Lys Leu
145                 150                 155                 160

Ala Asp Leu Thr Phe Leu Asp Val Ser Tyr Asn Arg Ile Pro Ser Ile
                165                 170                 175

Pro Met His His Ile Asn Leu Val Pro Gly Arg Gln Leu Arg Gly Ile
            180                 185                 190

Tyr Leu His Gly Asn Pro Phe Val Cys Asp Cys Ser Leu Tyr Ser Leu
        195                 200                 205

Leu Ile Phe Trp Tyr Arg Arg His Phe Ser Ser Val Met Asp Phe Lys
    210                 215                 220

Asn Asp Tyr Thr Cys Arg Leu Trp Ser Asp Ser Arg His Ser His Gln
225                 230                 235                 240

Leu Gln Leu Leu Gln Glu Ser Phe Leu Asn Cys Ser Tyr Ser Val Ile
                245                 250                 255

Asn Gly Ser Phe His Ala Leu Gly Phe Ile His Glu Ala Gln Val Gly
            260                 265                 270

Glu Arg Ala Ile Val His Cys Asp Ser Lys Thr Gly Asn Gly Asn Thr
        275                 280                 285

Asp Phe Ile Trp Val Gly Pro Asp Asn Arg Leu Leu Glu Pro Asp Lys
    290                 295                 300

Asp Met Gly Asn Phe Arg Val Phe Tyr Asn Gly Ser Leu Val Ile Glu
305                 310                 315                 320

Asn Pro Gly Phe Glu Asp Ala Gly Val Tyr Ser Cys Ile Ala Met Asn
                325                 330                 335

Arg Gln Arg Leu Leu Asn Glu Thr Val Asp Ile Met Ile Asn Val Ser
            340                 345                 350

Asn Phe Thr Ile Asn Arg Ser His Ala His Glu Ala Phe Asn Thr Ala
        355                 360                 365

Phe Thr Thr Leu Ala Ala Cys Val Ala Ser Ile Val Leu Val Leu Leu
    370                 375                 380

Tyr Leu Tyr Leu Thr Pro Cys Pro Cys Lys Cys Lys Ala Lys Arg Gln
385                 390                 395                 400

Lys Asn Thr Leu Ser Gln Ser Ser Ala His Ser Ser Ile Leu Ser Pro
                405                 410                 415

Gly Pro Thr Gly Asp Ala Ser Ala Asp Asp Arg Lys Ala Gly Lys Arg
            420                 425                 430

Val Val Phe Leu Glu Pro Leu Lys Asp Thr Ala Ala Gly Gln Asn Gly
        435                 440                 445

Lys Val Lys Leu Phe Pro Ser Glu Thr Val Ile Ala Glu Gly Ile Leu
    450                 455                 460

Lys Ser Thr Arg Ala Lys Ser Asp Ser Asp Ser Val Asn Ser Val Phe
465                 470                 475                 480

Ser Asp Thr Pro Phe Val Ala Ser Thr
                485

<210> 5
<211> 398
<212> PRT
<213> Homo sapiens

<400> 5
Met Ser Leu Arg Val His Thr Leu Pro Thr Leu Leu Gly Ala Val Val
1               5                   10                  15

Arg Pro Gly Cys Arg Glu Leu Leu Cys Leu Leu Met Ile Thr Val Thr
            20                  25                  30

Val Gly Pro Gly Ala Ser Gly Val Cys Pro Thr Ala Cys Ile Cys Ala
        35                  40                  45

Thr Asp Ile Val Ser Cys Thr Asn Lys Asn Leu Ser Lys Val Pro Gly
    50                  55                  60

Asn Leu Phe Arg Leu Ile Lys Arg Leu Asp Leu Ser Tyr Asn Arg Ile
65                  70                  75                  80

Gly Leu Leu Asp Ser Glu Trp Ile Pro Val Ser Phe Ala Lys Leu Asn
                85                  90                  95

Thr Leu Ile Leu Arg His Asn Asn Ile Thr Ser Ile Ser Thr Gly Ser
            100                 105                 110

Phe Ser Thr Thr Pro Asn Leu Lys Cys Leu Asp Leu Ser Ser Asn Lys
        115                 120                 125

Leu Lys Thr Val Lys Asn Ala Val Phe Gln Glu Leu Lys Val Leu Glu
    130                 135                 140

Val Leu Leu Leu Tyr Asn Asn His Ile Ser Tyr Leu Asp Pro Ser Ala
145                 150                 155                 160

Phe Gly Gly Leu Ser Gln Leu Gln Lys Leu Tyr Leu Ser Gly Asn Phe
                165                 170                 175

Leu Thr Gln Phe Pro Met Asp Leu Tyr Val Gly Arg Phe Lys Leu Ala
            180                 185                 190

Glu Leu Met Phe Leu Asp Val Ser Tyr Asn Arg Ile Pro Ser Met Pro
        195                 200                 205

Met His His Ile Asn Leu Val Pro Gly Lys Gln Leu Arg Gly Ile Tyr
    210                 215                 220

Leu His Gly Asn Pro Phe Val Cys Asp Cys Ser Leu Tyr Ser Leu Leu
225                 230                 235                 240

Val Phe Trp Tyr Arg Arg His Phe Ser Ser Val Met Asp Phe Lys Asn
                245                 250                 255

Asp Tyr Thr Cys Arg Leu Trp Ser Asp Ser Arg His Ser Arg Gln Val
            260                 265                 270

Leu Leu Leu Gln Asp Ser Phe Met Asn Cys Ser Asp Ser Ile Ile Asn
        275                 280                 285

Gly Ser Phe Arg Ala Leu Gly Phe Ile His Glu Ala Gln Val Gly Glu
    290                 295                 300

Arg Leu Met Val His Cys Asp Ser Lys Thr Gly Asn Ala Asn Thr Asp
305                 310                 315                 320

Phe Ile Trp Val Gly Pro Asp Asn Arg Leu Leu Glu Pro Asp Lys Glu
                325                 330                 335

Met Glu Asn Phe Tyr Val Phe His Asn Gly Ser Leu Val Ile Glu Ser
            340                 345                 350

Pro Arg Phe Glu Asp Ala Gly Val Tyr Ser Cys Ile Ala Met Asn Lys
        355                 360                 365

Gln Arg Leu Leu Asn Glu Thr Val Asp Val Thr Ile Asn Val Ser Asn
    370                 375                 380

Phe Thr Val Ser Arg Ser His Ala His Glu Ala Phe Asn Thr
385                 390                 395


<210> 6
<211> 22
<212> PRT
<213> Unknown Organism

<220>
<223> Description of Unknown Organism: Leucine zipper motif
      found in both human and mouse

<400> 6
Leu Tyr Leu Ser Gly Asn Phe Leu Thr Gln Phe Pro Met Asp Leu Tyr
  1               5                  10                  15

Val Gly Arg Phe Lys Leu
             20


<210> 7
<211> 33
<212> PRT
<213> Homo sapiens

<400> 7
Val Cys Pro Thr Ala Cys Ile Cys Ala Thr Asp Ile Val Ser Cys Thr
  1               5                  10                  15

Asn Lys Asn Leu Ser Lys Val Pro Gly Asn Leu Phe Arg Leu Ile Lys
             20                  25                  30

Arg

<210> 8
<211> 15
<212> PRT
<213> Homo sapiens

<400> 8
Ser Phe Ala Lys Leu Asn Thr Leu Ile Leu Arg His Asn Asn Ile
1               5                   10                  15


<210> 9
<211> 45
<212> PRT
<213> Homo sapiens

<400> 9
Thr Ser Ile Ser Thr Gly Ser Phe Ser Thr Thr Pro Asn Leu Lys Cys
1               5                   10                  15

Leu Asp Leu Ser Ser Asn Lys Leu Lys Thr Val Lys Asn Ala Val Phe
            20                  25                  30

Gln Glu Leu Lys Val Leu Glu Val Leu Leu Leu Tyr Asn
        35                  40                  45


<210> 10
<211> 24
<212> PRT
<213> Homo sapiens

<400> 10
Leu Lys Val Leu Glu Val Leu Leu Leu Tyr Asn Asn His Ile Ser Tyr
1               5                   10                  15

Leu Asp Pro Ser Ala Phe Gly Gly
            20


<210> 11
<211> 24
<212> PRT
<213> Homo sapiens

<400> 11
Leu Ser Gln Leu Gln Lys Leu Tyr Leu Ser Gly Asn Phe Leu Thr Gln
1               5                   10                  15

Phe Pro Met Asp Leu Tyr Val Gly
            20


<210> 12
<211> 25
<212> PRT
<213> Homo sapiens

<400> 12
Leu Ala Glu Leu Met Phe Leu Asp Val Ser Tyr Asn Arg Ile Pro Ser
1               5                   10                  15

Met Pro Met His His Ile Asn Leu Val
            20                  25


<210> 13
<211> 91
<212> PRT
<213> Homo sapiens

<400> 13
Phe Arg Ala Leu Gly Phe Ile His Glu Ala Gln Val Gly Glu Arg Leu
1               5                   10                  15

Met Val His Cys Asp Ser Lys Thr Gly Asn Ala Asn Thr Asp Phe Ile
            20                  25                  30

Trp Val Gly Pro Asp Asn Arg Leu Leu Glu Pro Asp Lys Glu Met Glu
        35                  40                  45

Asn Phe Tyr Val Phe His Asn Gly Ser Leu Val Ile Glu Ser Pro Arg
    50                  55                  60

Phe Glu Asp Ala Gly Val Tyr Ser Cys Ile Ala Met Asn Lys Gln Arg
65                  70                  75                  80

Leu Leu Asn Glu Thr Val Asp Val Thr Ile Asn
                85                  90

<210> 14
<211> 24
<212> DNA
<213> Artificial Sequence

<220>
<223> Description of Artificial Sequence: Synthetic
      primer

<400> 14
atgtcgttac gtgtacacac tctg                                              24

<210> 15
<211> 24
<212> DNA
<213> Artificial Sequence

<220>
<223> Description of Artificial Sequence: Synthetic
      primer

<400> 15
ttaagtggac gccacaaaag gtgt                                              24

<210> 16
<211> 25
<212> DNA
<213> Artificial Sequence

<220>
<223> Description of Artificial Sequence: Synthetic
      primer

<400> 16
aactcaggtc cagtctctta atcag                                             25

<210> 17
<211> 24
<212> DNA
<213> Artificial Sequence

<220>
<223> Description of Artificial Sequence: Synthetic
      primer

<400> 17
atgtcgttac gtgtacacac tctg                                              24

<210> 18
<211> 27
<212> DNA
<213> Artificial Sequence

<220>
<223> Description of Artificial Sequence: Synthetic
      primer

<400> 18
caaaatgaaa agacaggcaa acaaatg                                          27


<210> 19
<211> 39
<212> DNA
<213> Artificial Sequence

<220>
<223> Description of Artificial Sequence: Synthetic
      primer

<400> 19
atcctcgagg cgaccataat gtcgttacgt gtacacact                            39


<210> 20
<211> 36
<212> DNA
<213> Artificial Sequence

<220>
<223> Description of Artificial Sequence: Synthetic
      primer

<400> 20
gatcaccggt gcagtggacg ccacaaaagg tgtgtc                                36